(19) Europäisches Patentamt
European Patent Office
Office européen des brevets

(11) EP 2 764 457 B1

(12) EUROPEAN PATENT SPECIFICATION

(45) Date of publication and mention of the grant of the patent:
**12.12.2018 Bulletin 2018/50**

(21) Application number: **12768828.1**

(22) Date of filing: **04.10.2012**

(51) Int Cl.:
***G06F 19/16*** *(2011.01)* ***G06F 19/12*** *(2011.01)*

(86) International application number:
**PCT/EP2012/069636**

(87) International publication number:
**WO 2013/050481 (11.04.2013 Gazette 2013/15)**

# (54) METHOD OF EXPLORING THE FLEXIBILITY OF MACROMOLECULAR TARGETS AND ITS USE IN RATIONAL DRUG DESIGN

VERFAHREN ZUR UNTERSUCHUNG DER FLEXIBILITÄT VON MAKROMOLEKULAREN ZIELEN UND SEINE ANWENDUNG BEI RATIONALEM ARZNEIMITTELDESIGN

PROCÉDÉ D'EXPLORATION DE LA FLEXIBILITÉ DE CIBLES MACROMOLÉCULAIRES ET SON UTILISATION DANS UNE CONCEPTION RATIONNELLE DE MÉDICAMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2011 EP 11382313**

(43) Date of publication of application:
**13.08.2014 Bulletin 2014/33**

(73) Proprietors:
- **Universitat de Barcelona**
  **08028 Barcelona (ES)**
- **Barcelona Supercomputing Center-Centro Nacional de Supercomputación**
  **08034 Barcelona (ES)**
- **Fundació Institut de Recerca Biomèdica IRB (Barcelona)**
  **08028 Barcelona (ES)**
- **The University of Nottingham**
  **Nottingham NG7 2RD (GB)**

(72) Inventors:
- **OROZCO LÓPEZ, Modesto**
  **08769 Castellví De Rosanes (ES)**
- **LAUGHTON, Charles, Anthony**
  **Beeston**
  **Nottingham NG9 1FJ (GB)**
- **CARRILLO PARRAMÓN, Oliver**
  **08302 Mataró (ES)**
- **CHAUDHURI, Rima**
  **Darlinghurst, Sydney, NSW 2010 (AU)**

(74) Representative: **ZBM Patents - Zea, Barlocci & Markvardsen**
**Plaza Catalunya, 1**
**08002 Barcelona (ES)**

(56) References cited:
- **ARCANGELI C. ET AL: "Molecular dynamics simulation and essential dynamics study of mutated plastocyanin: structural, dynamical and functional effects of a disulfide bridge insertion at the protein surface", BIOPHYSICAL CHEMISTRY, vol. 92, no. 3, 18 September 2001 (2001-09-18), pages 183-199, XP055028827,**
- **HUANG S. Y. ET AL: "Ensemble docking of multiple protein structures: Considering protein structural variations in molecular docking", PROTEINS: STRUCTURE, FUNCTION, AND BIOINFORMATICS, vol. 66, no. 2, 1 February 2007 (2007-02-01), pages 399-421, XP055030637, ISSN: 0887-3585, DOI: 10.1002/prot.21214**
- **VAN DER SPOEL D. ET AL: "GROMACS: Fast, flexible, and free", JOURNAL OF COMPUTATIONAL CHEMISTRY, vol. 26, no. 16, 1 December 2005 (2005-12-01), pages 1701-1718, XP055028875, ISSN: 0192-8651, DOI: 10.1002/jcc.20291**
- **MONGAN J.: "Interactive essential dynamics", JOURNAL OF COMPUTER-AIDED MOLECULAR DESIGN, KLUWER ACADEMIC PUBLISHERS, DO, vol. 18, no. 6, 1 June 2004 (2004-06-01), pages 433-436, XP019248123, ISSN: 1573-4951**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 764 457 B1

- LEMMEN C & LENGAUER T: "Computational methods for the structural alignment of molecules", JOURNAL OF COMPUTER-AIDED MOLECULAR DESIGN, vol. 14, 2000, pages 215-232,
- AMADEI A ET AL: "Essential Dynamics of Proteins", PROTEINS: STRUCTURE, FUNCTION, AND GENETICS, vol. 17, 1993, pages 412-425,
- CHAKRAVORTY DK ET AL: "Energetics of Zinc-Mediated Interactions in the Allosteric Pathways of Metal Sensor Proteins", JOURNAL OF THE AMERICAN CHEMICAL SOCIETY, vol. 135, 7 December 2012 (2012-12-07), pages 30-33, DOI: 10.1021/ja309170g

## Description

The present invention relates to methods of computational drug design which are used to explore the flexibility of macromolecular targets. In particular, the method is related to ways of generating alternative structures of a macromolecular receptor corresponding to different physiologically relevant contexts with high computational efficiency, which opens up new venues for rational drug design.

BACKGROUND ART

The pharmaceutical industry is constantly trying to optimize the way drugs are designed. Currently, one of the most widespread paradigms being implemented in the search of New Molecular Entities (NMEs) is the so-called structure-based drug design. This approach is based on elucidating the structure of the macromolecular targets that play key roles in the development of disease, and based on those structures designing the therapeutic molecules that will interact with them, in order to achieve maximum complementarity and therefore potency and therapeutic benefit. Although the concept is simple, its application is fraught with problems, one of the key complicating factors being the flexibility of the receptors. Receptors are not rigid structures, they populate a very rich ensemble of conformations, and react differently to the presence of different changes in their environment. Therefore, in order to successfully apply structure-based drug design, not only the structure of the receptors is needed, but also their flexibility and deformation patterns (Teague SJ. "Implications of protein flexibility for drug discovery". Nat. Rev. Drug Disc. 2003, vol. 2, pp. 527-541).

Scientists gain insight into the structure of disease-associated receptors by application of several experimental techniques, X ray crystallography and Nuclear Magnetic Resonance (NMR) being the most important ones. These allow one to obtain the three-dimensional structure of the receptor of interest, either by itself or in complex with other molecules. Although these techniques have been evolving rapidly and are now routinely applied, they are not devoid of limitations. X ray crystallography requires crystals of the macromolecular target in question to obtain a proper diffraction pattern, and many proteins, globular and especially membrane-embedded ones, do not readily crystallize. Thus, in many instances the proteins must be genetically engineered, for example by mutation of some of their residues, to alter their behaviour and make them more soluble or more rigid. This is a trial and error process and no universal rules apply. Also, X-ray crystallography provides a static picture of the receptor or complex, so the deformability of the receptor is missed in the structure. It has also been argued that crystal packing effects might play in certain cases a non-negliggible role on the structure obtained (Davis AM., et. al. "Application and limitations of X-ray crystallographic data in structure-based ligand and drug design" Anqew. Chem. 2003, vol. 42, pp. 2718-2736). NMR on the other hand, gives access to a picture of the macromolecule in solution, and it is by nature a more flexible representation as not one but usually many structures of the same receptor are obtained that conform to the experimental restraints applied. However, this technique is currently limited by the size of the macromolecular receptor, which makes its application very challenging for many of the current pharmacologically interesting targets.

Shortcomings of current experimental techniques have prompted the application of theoretical methods on the study of structure and flexibility of the target receptors in search of complementary insight. There are numerous techniques that can be applied, amongst which molecular dynamics (MD) is widely regarded as the most rigorous and powerful one (Karplus M., et. al. "Molecular dynamics and protein function". Proc. of the Nat. Acad. of Sciences 2005, vol. 102, pp. 6679-6685). MD is a simulation technique in which molecules interact and evolve during a certain time window, giving a view of the motion of all partners involved along a timeline. The trajectories of these molecules are determined by numerically solving Newton's equations of motion, where the forces on each particle and the potetial energy of the system are defined by the force field. This simulation technique is applied to predict the dynamics of pharmacologically interesting targets in solution. Unfortunately, MD also suffers from limitations. It entails a heavy computational burden and the proper set up of the simulated systems is complex and requires high expertise, as does the analysis of trajectories once they have been obtained. Lastly, the technique suffers from not being able to overcome high conformational barriers in the potential energy surface of the receptors and complexes, thus being only able to explore ensemble conformations that are around the initial structures considered. As a result, MD is currently used to study with high accuracy specific protein-drug complexes, but it is far from being a high-throughput technique in theoretical drug design.

The application of further theoretical methods such as Normal Mode Analysis (NMA) and Essential Dynamics (ED) has recently opened up new ways to study receptor flexibility (Rueda M., et. al. "Thorough validation of protein normal mode analysis: Comparative study with essential dynamics". Structure 2007, vol. 15, pp. 565-575). These are powerful methods which operate on an equilibrium ensemble, and allow the separation of irrelevant local oscillations of the target receptor from concerted overall motions of a more collective type. Thus, their application gives access to the most important overall vibrational modes of deformation of a receptor. These collective motions, which usually have low frequency and high amplitude, have been found to be highly relevant both in terms of molecular recognition and biological function. Therefore, these analyses offer a filtered and more precise insight on the deformation patterns of receptors than a simple standard MD simulation.

The Essential Dynamics (ED) method can be applied to any equilibrium ensemble, be it from NMR, X-ray crystallography, or an MD trajectory. For ED, a Principal Component Analysis (PCA) is carried out on a large number of snapshots taken for instance from a MD simulation. The variance-covariance matrix of atomic fluctuations must be diagonalized once the rotation and translation of the receptor has been removed, to yield a number of eigenvectors and eigenvalues. The eigenvectors represent a new set of coordinates encoding the essential deformations of the overall structure of the receptor, each one in turn being orthogonal to the rest, i.e. the direction of each eigenvector is uncorrelated to the others, and their degree of fluctuation is given by their respective eigenvalues. Thus, the eigenvectors with bigger eigenvalues are the ones explaining the most of the variance in the receptor flexibility, i.e. the deformation patterns of most value, while those with very small eigenvalues describe uninteresing local oscillations. Remarkably, for many receptors of pharmacological interest, just the first few eigenvectors explain the majority of the variance found in the trajectory, i.e. the most of the overall deformation found for the receptor. This allows filtering the overall flexibility of the receptors into just a few collective degrees of freedom. These collective degrees of freedom have usually a very important role for the biological function of the receptor, and therefore represent a particularily attractive piece of information for the design of ligands. In fact, it has been recently seen that a MD trajectory can be successfully compressed and stored in the new set of ED coordinates, and by choosing only the first few most significant eigenvectors one can simplify the dynamics of the whole macromolecule, and later on back project the structures to cartesian coordinates with nearly no loss of accuracy (Meyer T., et. al. "Essential dynamics: A tool for efficient trajectory compression and management". J. Chem. Theory Comput. 2006, vol. 2, pp. 251-258). That is, by discarding many of the ED coordinates with minor eigenvalues, one can simplify the dynamic behaviour found in the ensemble of structures and still retain the most significant deformations of the macromolecular receptor.

In a further application, the ED technique has previously been used to enhance the sampling of a protein for which short MD simulations are available. Knowledge of the essential eigenvectors can be used to enhance sampling, by way of restraining the whole system in ED space, and guiding it along the principal eigenvectors (Amadei A. et. al. "An efficient method for sampling the essential subspace of proteins". J. Biomol. Str. Dyn. 1996, vol. 13, pp. 615-625). By carrying out this procedure, one can generate structures that sample a space defined by said eigenvectors more efficiently than by a standard MD technique. However, no methods based on ED have been developed to date to sample and access the conformational ensemble of a system closely related, but different, to the one for which the standard MD trajectory is available.

Although ED gives access to a richer picture of the flexibility, it also suffers from being computer and labour intensive, as is usually based on a pre-computed MD trajectory. Thus, in order to find out how the presence of a particular perturbation, such as the presence of a ligand or a mutation, changes the overall flexibility of a certain receptor in terms of its essential degrees of deformation, one must first run a standard MD simulation accounting for the perturbation, and then perform ED-PCA on its covariance matrix. This is by itself cumbersome, but in practice it is complicated even further, as standard MD is unable to jump over high energy barriers, and the effects of the addition of a change in the receptor under study, as is for example the addition of a mutation on the protein sequence, is not easily captured in the usual timescale of the MD simulations (currently on the order of hunderds of nanoseconds) (see, for example, Arcangeli C. et al., "Molecular dynamics simulation and essential dynamics study of mutated plastocyanin: structural, dynamical and functional effects of a disulfide bridge insertion at the protein surface", Biophysical Chemistry 2001, vol. 92, pp. 183-199). Thus, it would be highly desirable to have means to access the essential deformation pattern of a receptor in different situations (mutations, presence of ligands, etc), without the need to first calculate a standard MD trajectory for each one of them, and to be able to do so by a technique that allows overcoming high energy barriers on the potential energy surface in an easier way than with a standard MD simulation. This would grant access to a richer picture of flexibility in a more computer-efficient manner than is now currently available, and ultimately to a richer picture of the molecular recognition of the targets linked to disease.

## SUMMARY OF THE INVENTION

The scope of the invention is defined by the appended set of claims. The invention disclosed herein relates to a method (perturbed-EDMD) to generate a structural ensemble of a macromolecular receptor based on an ensemble of a closely related receptor, making use of a hybrid description, in which part of the system is expressed in ED space, and the perturbation is added in cartesian space. It can be used to have access to a dynamic picture of the receptor when under the influence of different perturbations. Therefore, contrary to what has been described in the prior art, perturbed-EDMD enables capturing the structure and flexibility of a receptor derivative (perturbed receptor) for which no MD trajectory is available, by making use of a MD trajectory of a closely related receptor (unperturbed). One of the advantages of this method is that the nature of the perturbation considered can be highly diverse, which allows its application to a whole range of contexts:

1) The perturbation can be the presence of a small molecule in the active site of a receptor such as a protein enzyme.

This allows to have a computationally efficient means to obtain the structure and deformation modes of a holo-enzyme when the structure of the apo-enzyme is available. In structure-based drug design, it is known that the structure of a receptor when binding a particular ligand (holo structure) is more useful for virtual screening purposes than the structure of that receptor when unbound (apo structure) (see Huang S. Y. et al., "Ensemble Docking of Multiple Protein Structures: Considering Protein Structural Variations in Molecular Docking" 2007, PROTEINS: Structure, Function, and Bioinformatics, vol. 66, pp. 399-421). This is because most active sites collapse, even if only slightly, in the absence of a small molecule binding to them. This slight collapse is many times enough to preclude binding of molecules due to steric clash. On the other hand, if a small molecule is present in the active site, and the latter is rearranged accordingly, some of the residues lining it are already pre-organized to facilitate binding, and so a virtual screening calculation is more likely to give successful results.

2) The perturbation introduced can also be the presence of a partner macromolecule binding to the original receptor under study. Thus, for example, if the structural ensemble of a protein is at hand, the method efficiently enables access to a new ensemble of the same protein when bound to another protein partner (whose structural ensemble is also pre-computed). This opens up a way to study allosteric modulation of certain targets. It is known that there can be changes in the active site of a receptor when other accessory macromolecules are binding to it, even in remote distant places, and that can have an impact on its molecular recognition.

3) Also, the perturbation on the original receptor system can be the presence of a post-translational modification. Proteins undergo numerous post-translational modifications once they are synthesized, ranging from the formation of disuphide bonds, through the glycosilation of some of their residues, phosphorylations on their serine, threonine and tyrosine residues, to proline isomerizations and many others. The method of the present invention allows efficient access to changes in the structure and deformation modes that some of the post-translational modifications can cause, if an ensemble of the unmodified protein is available.

4) Additionally, the perturbation can also be one or several point mutations in the receptor sequence. It is known that certain mutations, even in places remotely located with respect to the active site, can have a great impact on the molecular recognition of proteins and ligands. The method allows to efficiently find out the impact of one or several specific mutations on the structure of the receptor and its deformation modes. Thus, one of the most promising applications is in the area of mutations conferring resistance to certain drugs. Another application for the perturbing effects of mutations can be in the context of antigen-antibody recognition. It is known that some residues play a key role facilitating the deformation of antibodies and allowing for a natural fit with the antigen by acting as molecular hinges. The mutation of these residues can have direct consequences in the biological activity of such antibodies, and they can also be more easily studied by applying the method disclosed herein.

Additional advantages of the perturbed-EDMD method are: i) the fact that there is no need to compute a standard MD trajectory of the receptor for each one of the perturbations under study in order to have its essential modes of deformation, i.e. many of the perturbations on the same receptor can be accessed by processing a single standard MD simulation of the unperturbed receptor, and ii) the changes in structure and flexibility due to the perturbation are more easily captured than in a standard MD simulation, as the exploration is done on essential space, and thus high energy barriers on the potential surface of the perturbed receptor can more easily be overcome. This means, in practical terms, that changes in structure and flexibility due to drug binding, point mutations, and others, which would unlikely be captured by a short standard MD simulation, can be obtained in a computer efficient manner.

Thus, one aspect of the invention relates to a method of molecular modeling for generating alternative, perturbed structures of a macromolecular receptor from an ensemble of structures of the unperturbed receptor, comprising: a) aligning the ensemble of structures of the unperturbed receptor on a common reference frame to eliminate the rotational and translational degrees of freedom of the receptor; b) diagonalizing the covariance matrix of the positional fluctuations built with the ensemble of structures of the unperturbed receptor to obtain the eigenvectors and eigenvalues; c) defining a hybrid Hamiltonian of a perturbed state of the receptor in terms of a combination of the eigenvectors obtained in step b) plus a perturbation term in cartesian space (*V*) that accounts for the difference between the unperturbed state and the perturbed state

$$H = H^{(M)} + V$$

and; d) defining the new forces acting on the receptor under the influence of the perturbation

$$\vec{F}_i^{(M)} = -\sum_{k=1}^{M} \frac{k_B T e_k^i}{\lambda_k} \left(\Delta\vec{r} \cdot \hat{e}_i\right) + \vec{F}_i^*$$

where $k_B$ is Boltzmann's constant, $T$ is the absolute temperature, and where the sum on the right hand side is the resulting force derived from the Hamiltonian $H^{(M)}$, where $e_k^i$ is the component $i$ of the eigenvector corresponding to mode k, and integrating the equations of motion using Langevin or, alternatively, Brownian dynamics; and e) propagating the perturbed receptor to generate a trajectory and extracting a new ensemble of structures of the perturbed receptor.

In particular, it has been provided a method of molecular modeling for generating alternative, perturbed structures of a macromolecular receptor from an ensemble of structures of the unperturbed receptor, comprising

a) aligning the ensemble of structures of the unperturbed receptor on a common reference frame to eliminate the rotational and translational degrees of freedom of the receptor, by superimposing snapshots taken of the same macromolecule onto the common reference frame;
b) diagonalizing the covariance matrix of the positional fluctuations built with the aligned ensemble of structures of the unperturbed receptor to obtain the eigenvectors and eigenvalues;
c) defining mathematically a hybrid Hamiltonian of a perturbed state of the receptor as a combination of: c1) the eigenvectors obtained in step b), and
c2) a perturbation term in cartesian space that accounts for the difference between the unperturbed state and the perturbed state;
d) defining mathematically the resulting forces acting on the receptor under the influence of the perturbation term and integrating Newton's equations of motion using Langevin or, alternatively, Brownian dynamics; and
e) propagating the perturbed receptor by iteratively integrating Newton's equations of motion to generate a trajectory as in step d), and extracting a new ensemble of structures of the perturbed receptor.

Another aspect of the invention is described as the use of the ensemble of structures of the perturbed macromolecular receptor obtained by the method described above for virtual screening purposes. This aspect of the invention can also be formulated as a method for virtual screening comprising carrying out the method according to any of the embodiments and the subsequent use of the ensemble of structures for the perturbed macromolecular receptor thus obtained.

Another aspect of the invention relates to a computer program product comprising program instructions for causing a computer to perform the method of molecular modelling for generating alternative, perturbed structures of a macromolecular receptor as defined above. Said computer program may be embodied on storing means (for example, on a record medium, on a computer memory or on a read-only memory) or carried on a carrier signal to be, for example, downloaded from a computer or sent by an email (for example, on an electrical or optical carrier signal).

Another aspect of the invention relates to a system of molecular modeling adapted for performing the method as defined in the first aspect of the invention, the system comprising: a) computer means for aligning the ensemble of structures of the unperturbed receptor on a common reference frame to eliminate the rotational and translational degrees of freedom of the receptor;

b) computer means for diagonalizing the covariance matrix of the positional fluctuations built with the aligned ensemble of structures of the unperturbed receptor to obtain the eigenvectors and eigenvalues; c) computer means for defining a hybrid Hamiltonian of a perturbed state of the receptor in terms of a combination of the eigenvectors obtained in step b) plus a perturbation term in cartesian space that accounts for the difference between the unperturbed state and the perturbed state; d) computer means for defining the new forces acting on the receptor under the influence of the perturbation and integrating the equations of motion using Langevin or alternatively Brownian dynamics; and e) computer means for propagating the perturbed receptor to generate a trajectory and extracting a new ensemble of structures of the perturbed receptor.

It is important to highlight that the described system can be a part (for example, hardware in the form of a PCI card) of a computer system (for example a personal computer). On the other hand, the system can be an external hardware connected to the computer system by appropiate means.

BRIEF DESCRIPTION OF THE DRAWINGS

FIG. 1. a-d shows the enrichment plots for all the proteins considered as targets for virtual screening in search of new active compounds. The PDB identities found in Table I are on top of each graph for correspondance. In the x axis, the % of the database screened can be found. Only the top 15% of the screened databases was analyzed in each case, and it was fragmented in 4 bins corresponding to the top 1%, 5%, 10% and 15% of the database. In the y axis, the normalized number of True Positives (% of TPs) found in each bin is represented for the docked ranked list of ligands, for the three methods compared.

FIG. 2. The system 1UOR is seen in both pictures. (a) Represents the docked conformation of the active compound warfarin in one of the perturbed-EDMD snapshots. Ensemble docking in this case ranked the compound in the top 10% bin. The image clearly shows there is room for the compound to properly bind the interaction site. (b) Represents the docked conformation of the same compound in one of the ensemble MD snapshots. Ensemble docking in this case did not prioritize the compound in the top 15%. The image clearly shows there is no room for the compound to properly bind, as the conformation of the protein is not conducive to selecting warfarin as a top binder, and a steric clash against the protein is found and depicted by an arrow.

FIG. 3. The system 1A9U is seen in both pictures. (a) Represents one of the active compounds for the target (p38 MAP kinase) in its top ranking binding mode coming from the MD ensemble docking calculations. None of the interactions found for this compound in its true binding mode are found, and it is not found in the top 15% of the database. (b) Represents the same compound in its top ranking binding mode coming from the perturbed-EDMD ensemble docking. Basically all the interactions found in its true binding mode with the target are found, and it is prioritized in the top 5% of the database (the main interaction can be found depicted as dotted lines, which is a hydrogen bond with the hinge segment of the kinase)

DETAILED DESCRIPTION OF THE INVENTION

Definitions:

The terms used in the present application relate to computational chemistry and molecular modeling, and are well-known to the person skilled in the art. However, some definitions have been included in order to avoid any misinterpretation.

The term "aligning the ensemble of structures" as used herein means to superimpose all the snapshots taken of the same macromolecule on a common reference frame, so that the rotational and translational motions of the macromolecule are eliminated. In this way, a better picture of the positional fluctuations of each atom of the macromolecule about an average structure is accessible. This is normally done by a least squares fit of each of the snapshots onto the common reference frame.

The term "classical system" as used herein denotes a system where the atoms are represented as points in space. A classical representation is the alternative to a quantum mechanical representation where nuclear and electron positions are taken into account.

The term "common reference frame" as used herein describes a snapshot of the receptor system that is used as reference, such that all other snapshots are overlapped with it, in order to eliminate the movement of the receptor due to rotation over itself and translation due to drifting in the solvent.

The term, "defining mathematically a hybrid Hamiltonian" as used herein means to define the perturbed receptor as found in equation (2) found below.

The term "diagonalizing the covariance matrix" as used herein means to express the covariance matrix (C) which contains, as elements, the covariances of the atomic displacements relative to their respective averages for each pair of atoms for the off-diagonal elements and the variances of each atom displacements along the diagonal, in a simpler way, such that:

$$C=P\,D\,P^{-1}$$

where P is an invertible matrix and D is a diagonal matrix.

The term "ensemble" as used herein describes a set of different structures corresponding to a particular molecule that taken together represent the accessible configurational space the molecule explores under certain conditions of temperature, solvent used, interacting molecules present in the solution, etc.

The term "equations of motion" as used herein means the equations that describe the behaviour of a physical system in terms of its motion as a function of time, i.e. in terms of dynamic variables. Normally, spacial coordinates and time are used.

The term "equilibration" as used herein denotes a multi-step process by which the simulated system is taken from its initial state after building to a point where the system is ready for unrestrained molecular dynamics. The process typically implies starting with low temperatures (for instance 100K) and tight restraints on a certain part of the system, usually the solute. The temperature is then gradually increased to reach room temperature, and the restraints are gradually removed so that the system is more and more free to move away from its initial position.

The term "force field" as used herein denotes a set of equations that define the potential energy of the system as a function of atomic coordinates, and which are based on the use of parameters defining bond lengths, angles, dehidral angles, atom charges, van der Waals radii, etc, derived from experimental data and quantum mechanical calculations that allow representing a system in a classical way as opposed to quantum mechanically, where the representation of the system requires solving the wavefunction.

The term "MD average structure" as used herein denotes the structure of the receptor that is generated as a mean structure representing an ensemble extracted from a MD trajectory.

The term "minimization" as used herein denotes a calculation in which the atomic positions are optimized in order to find a local or a global minimum in the potential energy surface of a molecule.

The term "perturbation" as used herein denotes an element acting on the receptor in such a way that its structure and flexibility are, even if only locally, (partly) modified with respect to the unperturbed receptor. A perturbation then can be the mutation of certain amino acids in the sequence of a protein, the presence of a small organic molecule inside the active site of an enzyme, the presence of a post-translational modification on a protein such as the phosphorylation on a residue, or the non-covalent interaction of an auxilliary protein on the surface of a receptor.

The term "positional fluctuations", referring to an atom, as used herein means the movements of the atom around an average position. Atoms in molecules are always in motion due to temperature. For a molecule which has reached equilibrium and does neither translate nor rotate, the atoms fluctuate around an average position.

The term "propagating the perturbed receptor" as used herein means that once the forces acting on the atoms of the macromolecule under the influence of the perturbation have been computed, the integration of the equations of motion must be carried out in order to obtain a new set of positions after a time step. The atoms in the new positions are again under a set of new forces, which after a second time step will generate a new set of positions, etc. This cycle is repeated iteratively for as many time steps as needed, until the desired simulation time is reached.

The term "restraint" as used herein denotes a force exerted on an atom, a bond, an angle between 3 linked atoms, etc, that prevents it from deviating from a certain value. Restraints are used for example in the equilibration process of a molecular system, to ensure that the deviation with respect to the initial reference positions is not substantial. The stronger the force exerted, the less the atom location, bond, angle, etc. deviates from its original value.

The term "snapshot" as used herein denotes the picture of the molecular system at a certain point in time. It has the position and velocities of all atoms in a classical system.

The term "trajectory" as used herein denotes the history of the positions and motions (velocities) of all atoms in the system under study, over a certain period of time.

As mentioned above it has been provided a method of molecular modeling for generating alternative, perturbed structures of a macromolecular receptor from an ensemble of structures of the unperturbed receptor, a particular embodiment of the method comprises

a) aligning the ensemble of structures of the unperturbed receptor on a common reference frame to eliminate the rotational and translational degrees of freedom of the receptor, by superimposing snapshots taken of the same macromolecule onto the common reference frame;
b) diagonalizing the covariance matrix of the positional fluctuations built with the aligned ensemble of structures of the unperturbed receptor to obtain the eigenvectors and eigenvalues;
c) defining mathematically a hybrid Hamiltonian of a perturbed state of the receptor as a combination of: c1) the eigenvectors obtained in step b), and c2) a perturbation term in cartesian space that accounts for the difference between the unperturbed state and the perturbed state;
d) defining mathematically the resulting forces acting on the receptor under the influence of the perturbation term and integrating Newton's equations of motion using Langevin or, alternatively, Brownian dynamics; and
e) propagating the perturbed receptor by iteratively integrating Newton's equations of motion to generate a trajectory as in step d), and extracting a new ensemble of structures of the perturbed receptor.

In a particular embodiment, the aligning of the ensemble of structures of the unperturbed receptor on a common reference frame to eliminate the rotational and translational degrees of freedom of the receptor is typically, done by applying least squares fit. To do so, in our case, the ptraj module of the AMBER suite of programs was used. A prototypical

input file such as the one used to carry out the invention has the form of:

```
#!/bin/tcsh -f
set pdb=UBQ [name of the protein]
/hosts/pluto/soft/amber9/amber9/exe/ptraj UBQ.top [topology file of the
protein] <<EOF
trajin snapshot1.pdb
trajin snapshot2.pdb
trajin snapshot3.pdb
trajin snapshot4.pdb
[list of all the snapshots we would like to analyse]
trajin snapshot1000.pdb
reference snapshot1 .pdb
rms reference
matrix covar name covar mass
analyze matrix covar out UBQ.evec vecs 1000 [number of snapshots]
atomicfluct * out UBQ.bfactor byres bfactor
go
EOF
```

The rms command is the one responsable for aligning all the snapshots with respect to the "reference" snapshot.

In another particular embodiment, the diagonalization of the covariance matrix of the positional fluctuations built with the ensemble of structures of the unperturbed receptor to obtain the eigenvectors and eigenvalues is done with the ptraj module of the AMBER suite of programs, by using the "analyze matrix covar out" instruction (see input file found above). This gives the eigenvectors and eigenvalues in the file UBQ.evec (following the example of point a)).

In another particular embodiment, the definition of a hybrid Hamiltonian of a perturbed state of the receptor is the sum of two parts. On the one hand, the ED/MD potential defined in equation (1) (see below) which is defined by using the results of the previous step, and on the other a potential that accounts for the interaction between the receptor and another physical system interacting with it (the perturbation).

In another particular embodiment the new forces of step d) are computed analitically using formula (4) found below, and once they are computed, they are used to integrate Newton's equations of motion in the Langevin dynamics or alternatively Brownian dynamics approaches.

In another particular embodiment, the equation of motion is equation (S1)

Typically, the integration of this equation of motion is carried out by using the Verlet algorithm with stochastic integrals, as found in equations (S4) and (S5).

In the abovementioned method, the propagation carried out in step d) refers to iteratively integrating Newton's equations of motion until a simulation time is reached.

Any combination of the particular embodiments mentioned above are also part of the invention.

In a preferred embodiment of the invention, the perturbation term corresponds to the addition of an extra molecule binding on a certain point of the receptor.

In another preferred embodiment of the invention, the extra molecule is a protein. By adding a protein as a perturbation to the original system, one opens up the possibility of studying allosteric mechanisms among macromolecules. If the unperturbed receptor is a protein whose conformational ensemble is known, the application of the method of the present invention allows accessing the conformational ensemble of the protein when bound to other auxilliary proteins that could modulate its activity or change the preferred conformation of its active site residues, which could have an impact on the way it interacts with synthetic molecules.

In another preferred embodiment of the invention, the extra molecule is an organic molecule. In enzyme inhibitor design, oftentimes only the structure of the enzyme is available (apo structure) without the presence of a substrate or inhibitor. Although this is by itself precious information, the fact that the active site of the receptor is not occupied can have deleterious consequences when the structure is used for virtual screening purposes. Most active sites cave in when there are no substrates or inhibitors in their inside, and this precludes the *in silico* structure-based screening of libraries, as most small molecules clash against the residues lining the active site. The method presented herein represents a new way of accommodating an active site so that it is expanded and therefore more ready to interact with small synthetic molecules.

In another preferred embodiment of the invention, the perturbation term corresponds to the addition of one or several mutations on the receptor. By being able to generate different ensembles of the same protein due to the addition of different point mutations in its aminoacid sequence (using the same unperturbed ensemble), the method opens up the possibility of studying the effects of mutations in a more high throughput manner. This has direct implications for example in the area of antigen-antibody recognition, as it is known that some amino acids play key roles acting as hinges

between different subunits of the same polypeptide, such as the different Ig-like domains of an antibody. The effects such mutations can have in the flexibility (and in turn activity) of the whole macromolecule can be more easily studied by the application of the method disclosed herein.

In another preferred embodiment of the invention, the perturbation term corresponds to the addtion of post-translational modifications of the receptor. Proteins, which are the usual targets in pharmacology, can undergo (once synthesized) many post-translational changes that can have an impact on their structure, their function and therefore on their molecular recognition and interaction with pharmacological agents. By applying the method presented herein, one can have access to the structure and devise changes in the main deformation modes of a post-translationally modified protein by using an ensemble of the unmodified protein.

In another preferred embodiment of the invention, the post-translational modification perturbing the receptor is the phosphorylation of one or more of its tyrosine, threonine or serine residues. Phosphorylation is an ubiquitous mechanism exploited by nature to knock up or down different signals at the cellular level by activating or inhibiting certain proteins. Thus, for many proteins, conformational changes are brought about after being phosphorylated, and these are important in the design of inhibitors. The method presented herein can be used to quickly find out about the impact this important modification can have on proteins of pharmacological interest.

In another preferred embodiment of the method of molecular modeling of the invention, the process further comprises a previous step where molecular dynamics simulations are used to obtain the ensemble of structures of the unperturbed receptor.

In another preferred embodiment of the method of molecular modeling of the invention, the process further comprises a previous step where Montecarlo simulations are used to obtain the ensemble of structures of the unperturbed receptor.

In another preferred embodiment of the method of molecular modeling of the invention, the process further comprises a previous step where Nuclear Magnetic Resonance or X-ray crystallography are used to obtain the ensemble of structures of the unperturbed receptor.

In another preferred embodiment of the method of molecular modeling of the invention, the method further comprises the steps of: f) docking each small molecule found in a database of small organic molecules to at least one structure of the ensemble of structures of the perturbed macromolecular receptor; g) estimating the affinity of each docked small molecule to the at least one structure of the ensemble of structures of the perturbed macromolecular receptor by calculating the interaction energy between the docked small molecule and the receptor, or alternatively by calculating the binding enthalpy or alternatively by calculating the binding free energy; and h) ranking all the small molecules found in the database of small organic molecules based on the results of step g).

In yet another preferred embodiment of the method of molecular modeling of the invention, the estimation of the affinity is carried out by calculating the binding free energy wherein the calculation comprises the use of a scoring function.

This last embodiment can be formulated as a method of virtual screening of a database of small organic molecules with a perturbed structure of a macromolecular receptor comprising:

a) aligning the ensemble of structures of the unperturbed receptor on a common reference frame to eliminate the rotational and translational degrees of freedom of the receptor;
b) diagonalizing the covariance matrix of the positional fluctuations built with the ensemble of structures of the unperturbed receptor to obtain the eigenvectors and eigenvalues;
c) defining a hybrid Hamiltonian of a perturbed state of the receptor in terms of a combination of the eigenvectors obtained in step b) plus a perturbation term in cartesian space that accounts for the difference between the unperturbed state and the perturbed state;
d) defining the new forces acting on the receptor under the influence of the perturbation and integrating the equations of motion using Langevin or alternatively Brownian dynamics;
e) propagating the perturbed receptor to generate a trajectory and extracting a new ensemble of structures of the perturbed receptor;
f) docking each small molecule found in a database of small organic molecules to at least one structure of the ensemble of structures of the perturbed macromolecular receptor;
g) calculating the free energy of binding of each docked small molecule to the at least one structure of the ensemble of structures of the perturbed macromolecular receptor by using a scoring function; and
h) ranking all the small molecules found in the database of small organic molecules based on the results of step g).

In a particular embodiment, the docking is carried out by optimizing the interaction of each small molecule found in the database with the receptor by finding the best pose inside the active site by using a fitness function such as the one found in the Glide program (Schrodinger Inc.).
In another particular embodiment, the calculation of the free energy of binding of the docked small molecule to the

perturbed macromolecular receptor is carried out by using a scoring function such as the one found in the Glide program (Schrodinger Inc.).

Any combination of the particular embodiments mentioned above are also part of the invention.

The method of the present disclosure is based on the fact that the trajectory of a receptor can be inferred from previously computed trajectories of a closely related receptor. For example, the dynamics of a particular protein-ligand complex can be inferred from the dynamics of the complex of the same protein with another ligand. The starting point can be an atomistic MD simulation from which an ensemble of conformations of the macromolecule $\langle r \rangle$ is obtained. Alternatively, the ensemble can also be obtained by experimental means, such as Nuclear Magnetic Resonance. This in turn defines a covariance matrix, whose diagonalization yields a set of (3N-6) eigenvectors ($\{\hat{e}_i\}$), describing the nature of the essential deformation movements, and the associated set of eigenvalues ($\{\lambda_i\}$) determining the magnitude of the deformation sampled in each deformation movement (being N the number of atoms of the system considered). It has been seen that a (Cartesian coordinate) trajectory can be projected into the entire set of eigenvectors with no loss of accuracy. Furthermore, an approximated trajectory can be obtained by a back transformation that only considers projections along the most important eigenvectors (those explaining the largest amount of variance in the trajectory either for the entire receptor or fragments of it, for example the binding site of an enzyme). Within the harmonic limit, the movements of the macromolecule along the essential movements can be represented by an effective Hamiltonian:

$$H^{(M)} = \sum_{i=1}^{3N} \frac{p_i^2}{2m_i} + \frac{1}{2}\sum_{i=1}^{M} \frac{k_B T}{\lambda_i}\left(\Delta\vec{r} \cdot \hat{e}_i\right)^2 \qquad (1)$$

where i stands for a particle, $p_i$ is the i-th component of the momentum vector, $m_i$ the particle mass, $\Delta\overline{r} = \overline{r} - \overline{r}_0$ is the displacement from the equilibrium position of atoms $(\overline{r}_0)$,$k_B$ is Boltzmann's constant and $T$ is the absolute temperature. It is worth noting that this Hamiltonian can be evaluated to a given level of accuracy by simply restricting the sum to the M most significant deformation modes (instead of considering all the 3N-6 modes). The restriction of the eigenvector space (M< 3N-6) allows one to concentrate the computational effort on relevant modes and to use large integration time steps. It should be noted that it is also possible to restrict the Hamiltonian to a given set of atoms or to reproduce a part of the molecule at the coarse-grained level (for example at the $C_\alpha$ level in case the receptor is a protein), keeping all the atomistic detail in another region. In both cases the part of interest is reproduced with the same detail as in the original unrestricted-fully atomistic trajectory.

For a system according to the invention where interactions other than those from the original MD simulation exist, an effective Hamiltonian can be defined by adding a perturbation term ($V$), expressed in Cartesian terms:

$$H = H^{(M)} + V \qquad (2)$$

The corresponding perturbational 3N-force $\overline{F} = -\overline{\nabla} V$ acting on the protein ($\overline{\nabla} = (\partial/\partial r_1,...,\partial/\partial r_{3N})$) is reduced to avoid divergences due to higher mode contributions, leading to a reduced force:

$$\vec{F}^* = \vec{F} - \sum_{k=M+1}^{3N} \varphi_k \hat{e}_k \qquad (3)$$

where $\{\varphi_1,...,\varphi_M,\varphi_{M+1}...,\varphi_{3N}\}$ are the components of $\overline{F}$ in the base $\hat{e}$.

The resulting force acting on the macromolecule under the influence of a perturbational potential is then computed as:

$$\vec{F}_i^{(M)} = -\sum_{k=1}^{M} \frac{k_B T e_k^i}{\lambda_k}\left(\Delta\vec{r} \cdot \hat{e}_i\right) + \vec{F}_i^* \qquad (4)$$

where the sum on the right hand side is the resulting force derived from the Hamiltonian $H^{(M)}$, where $e_k^i$ is the component $i$ of the eigenvector corresponding to mode $k$. The integration of the equations of motions derived from eq. 4 is performed using Langevin's procedure as explained below. Integration of equations of motion. The effective forces computed using eq. 4 define a Langevin equation which needs to be integrated to obtain new trajectories:

$$m_i \ddot{\vec{r}}_i = \vec{F}_i^{(M)} - \gamma \dot{\vec{r}}_i + \vec{\xi}_i^* \qquad \text{(S1)}$$

where $\gamma$ stands for a friction coefficient and Stochastic terms should satisfy:

$$\left\langle \vec{\xi}_i^*(t) \right\rangle = 0$$

$$\left\langle \vec{\xi}_i^*(t) \vec{\xi}_j^*(t') \right\rangle = 2k_B T^* \gamma \delta_{ij} \delta(t-t') \qquad \text{(S2)}$$

and the effective temperature ($T^*$) is defined as:

$$T^* = \frac{3N}{M} T \qquad \text{(S3)}$$

Numerical integration is done using Verlet algorithm:

$$\vec{r}_i = \vec{r}_i^{\,0} + \tau\left(1 - e^{-\Delta t/\tau}\right)\vec{v}_i^{\,0} + \frac{\Delta t}{\tau}\left(1 - \frac{\tau}{\Delta t}\left(1 - e^{-\Delta t/\tau}\right)\right)\vec{F}_i^{\,0} + \Delta\vec{r}_i^{\,G} \qquad \text{(S4)}$$

And

$$\vec{v}_i = e^{-\Delta t/\tau}\vec{v}_i^{\,0} + \frac{1}{\gamma}\left(1 - e^{-\Delta t/\tau}\right)\vec{F}_i^{\,0} + \Delta\vec{v}_i^{\,G} \qquad \text{(S5)}$$

with stochastic integrals:

$$\Delta\vec{r}_i^{\,G} = \frac{1}{\gamma m_i}\int_t^{t+\Delta t}\left[1 - e^{-\gamma(t+\Delta t - t')}\right]\vec{\xi}_i^*(t')dt' \qquad \text{(S6)}$$

$$\Delta\vec{v}_i^{\,G} = \frac{1}{m_i}\left[e^{-\gamma(t+\Delta t - t')}\vec{\xi}_i^*(t')\right]dt' \qquad \text{(S7)}$$

In order to avoid excitation of the negligible modes, the stochastic term $\overline{\xi^*}$ is truncated summing up to the *M*-th mode of the exact noise vector term:

$$\vec{\xi}^* = \vec{\xi} - \sum_{k=M+1}^{3N} \rho_k \hat{e}_k \qquad \text{(S8)}$$

where $\{\rho_1, ..., \rho_M, \rho_{M+1}, ..., \rho_{3N}\}$ are the components of the noise vector $\overline{\xi}$ in the eigenvector base $\hat{e}$.

Trajectories are obtained by activating movements along a set of essential deformations, representing movements with different associated frequencies. The optimum integration time step ($\Delta t$) can be determined for each mode based on its characteristic frequency:

$$\Delta t_i < 2\pi\left(\frac{m_s \lambda_i}{k_B T}\right)^{1/2} \qquad \text{(S9)}$$

where $\Delta t_i$ is the time step associated to the integration of movements along mode $i$ , $\lambda_i$ is its eigenvalue, T is the non-reduced temperature and $m_s$ is the mass of the smallest particle (to accelerate calculations and avoid divergences hydrogen trajectories are not integrated and accordingly $m_s$ corresponds to the carbon mass).

The use of multiple time steps, which accelerates calculation, can be made evident by rewriting eq. S1 as:

$$m_i\ddot{\vec{r}}_i = -\sum_{k=1}^{M}\frac{k_B T e_k^i}{\lambda_k}\left(\Delta\vec{r}\cdot\hat{e}_k\right)+\vec{F}_i^* - \gamma\dot{\vec{r}}_i + \vec{\xi}_i^* \qquad \text{(S10)}$$

where the first term in right hand part of equation S10 accounts for the internal force of the protein and the second term is the external force. If we divide both sides by $m_i$ and develop the dot product of the sum, we get,

$$\ddot{\vec{r}}_i = -k_B T\sum_{j=1}^{3N}\left(\sum_{k=1}^{M}\frac{e_k^j e_k^j}{m_i\lambda_k}\right)\Delta\vec{r}_j + \frac{\vec{F}_i^*}{m_i} - \frac{\gamma}{m_i}\dot{\vec{r}}_i + \frac{\vec{\xi}_i^*}{m_i} \qquad \text{(S11)}$$

which allows us to define a set of frequencies as

$$\omega_{ij}^2 = \sum_{k=1}^{M}\frac{e_k^j e_k^j}{m_i\lambda_k} \qquad \text{(S12)}$$

and rewrite Eq.S11 as:

$$\ddot{\vec{r}}_i = -k_B T\sum_{j=1}^{3N}\omega_{ij}^2\Delta\vec{r}_j + \frac{\vec{F}_i^*}{m_i} - \frac{\gamma}{m_i}\dot{\vec{r}}_i + \frac{\vec{\xi}_i^*}{m_i} \qquad \text{(S13)}$$

By defining a threshold frequency $\omega_0$, modes can be divided into fast ($\omega_{jk} \geq \omega_0$) and slow (($\omega_{jk} \leq \omega_0$) so that the sum in eq. S13 can be rewritten as:

$$\sum_{j=1}^{3N}\omega_{ij}^2\Delta\vec{r}_j = \sum_{\omega\geq\omega_0}\omega_{ij}^2\Delta\vec{r}_j + \sum_{\omega\leq\omega_0}\omega_{ij}^2\Delta\vec{r}_j \qquad \text{(S14)}$$

Integrations along fast motions need to be done frequently, but slow modes can be integrated less often, adding their contribution to the displacement along fast modes as a perturbation ($\overline{\delta_i}(t)$) which is re-evaluated every *n*-steps (i.e. when we consider that slow forces need to be re-evaluated):

$$\vec{r}_i(t) = \vec{r}_i^{(0)}(t) + \vec{\delta}_i(t) \qquad \text{(S15)}$$

where $\overline{r_i}^{(0)}(t)$ is the fast modes solution, which have to be evaluated at each time step $\Delta t$. Substituting Eq. S15 in Eq. S13, we get an acceleration equation concerned to coordinate $i$ at time t:

$$\ddot{\vec{r}}_i(t) = \ddot{\vec{r}}_i^{(0)} + \ddot{\vec{\delta}}_i \qquad \text{(S16)}$$

from which we can distinguish the contribution of the fast and slow motion terms:

$$\ddot{\vec{r}}_i^{(0)} = -k_B T \sum_{fast} \omega_{ij}^2 \Delta\vec{r}_j^{\,0} + \frac{\vec{F}_i^*}{m_i} - \frac{\gamma}{m_i}\dot{\vec{r}}_i^{(0)} + \frac{\vec{\xi}_i^*}{m_i}$$

$$\ddot{\vec{\delta}}_i = -k_B T \sum_{slow} \omega_{ij}^2 \Delta\vec{r}_j^{\,0} - k_B T \sum_{j=1}^{3N} \omega_{ij}^2 \vec{\delta}_j - \gamma\,\dot{\vec{\delta}}_i \qquad \text{(S17)}$$

It must be noted that the method can be generalized to several different intervals for which integration is done at different time steps.

The method of the present invention can be implemented for the study of protein-ligand interactions. As presented above the method is general and can be tuned to a different scenarios. For example, in the case of a drug we can introduce a perturbational potential (see eq. 2) accounting for solvation (sol), non-bonded (van der Waals (vw) and electrostatic (ele)) interactions:

$$H = H_{prot}^{(M)} + H_{lig} + V_{ele}\left(\vec{d}_{ij}, \varepsilon_{ij}\right) + V_{vW}\left(\vec{d}_{ij}\right) + V_{solv}\left(\vec{d}_{ij}\right) \qquad \text{(S18)}$$

with

$$H_{lig} = \sum_{j=1}^{3N_{lig}} \frac{p_{lig,j}^2}{2m_{lig,j}} + V_{lig}\left(\vec{d}_{jk}^{\,\text{int}}\right) \qquad \text{(S19)}$$

where $\overline{d}_{ij}$ is the vector distance between atom i of the protein and atom j of the drug. $N_{tig}$ is the number of atoms of the drug, $p_{lig,j}$ is the *j*-th component of its momentum vector and $m_{lig,j}$ is its corresponding mass. $V_{lig}$ is the potential energy of the atoms of the drug due to their internal interactions and $\vec{d}_{jk}^{\,\text{int}}$ is the intramolecular vector distance between atom *j* and atom *k* in the drug.

The related perturbation force is computed as:

$$\vec{F}_i = -\vec{\nabla}_i V_{ele}\left(\vec{d}_{ij}, \varepsilon_{ij}\right) - \vec{\nabla}_i V_{vw}\left(\vec{d}_{ij}\right) - \vec{\nabla}_i V_{sol}\left(\vec{d}_{ij}\right) \qquad \text{(S20)}$$

while for drug atoms forces are computed as:

$$\vec{F}_j = -\vec{\nabla}_j V_{ele}\left(\vec{d}_{ij}, \varepsilon_{ij}\right) - \vec{\nabla}_j V_{vw}\left(\vec{d}_{ij}\right) - \vec{\nabla}_j V_{sol}\left(\vec{d}_{ij}\right) - \vec{\nabla}_j V_{lig}\left(\vec{d}_{jk}\right) \qquad \text{(S21)}$$

where the electrostatic forces are computed using Mehler and Solmajer formalism (Mehler EL. et. al. "Electrostatic effects in proteins: comparison of dielectric and charge models" Protein Eng. 1991, vol. 4, pp. 903-910), van der Waals is represented using a soft potential and solvation is accounted for by means of Lazaridis-Karplus functional (Lazaridis, T. et. al. "Effective energy function for proteins in solution" Proteins 1999, vol. 35, pp. 133-152), or any other alternative solvation method. It should be noted that perturbational forces modulate protein motion, so the approach captures ligand-induced changes in protein dynamics.

The method of the present invention can also be implemented for protein-protein interactions. The method outlined above can be generalized to the motion of a protein A in the field of another one B to simulate for example their relative diffusion or direct interaction by using:

$$H = H_A^{(M_A)} + H_B^{(M_B)} + V_{AB}\left(\vec{d}_{ij}\right) \qquad \text{(S22)}$$

This Hamiltonian accounts for the unperturbed Hamiltonian of both proteins ( $H_A^{(M_A)}$ and $H_B^{(M_B)}$ ) up to a

given degree of accuracy ($M_A$ for protein A and $M_B$ for protein B) and the interaction potentials $V_{AB}$ which are defined either as the sum of a soft van der Waals term, electrostatic and solvation energies or as a coarse-grained potential, which can be expressed using either physical or knowledge-based potentials.

The method of the present invention can also be implemented for the study of mutated proteins. Within the method this is done by replacing at each mutant site the "*r*" atoms from the wild type protein with the "*s*" new atoms of the mutated amino acid. The MD-average structure of the wild type is taken as reference, standard residue libraries and relaxation protocols are used to refine the starting model of the mutated protein. Trajectory for the wild type is then obtained by using the effective Hamiltonian:

$$H = H_{<}^{(M)} - H_{prot-r} + H_s + H_{prot-s} \qquad \text{(S23)}$$

where $H_{<}^{(M)}$ is the basic Hamiltonian defined above without the contributions of the removed atoms, $H_{prot\text{-}r}$ is the energy due to the interactions between removed atoms (r) and the rest of the protein, $H_s$ is intra-molecular Hamiltonian of the added mutation atoms (s), and $H_{prot\text{-}s}$ reproduces the interactions between added atoms and the protein. All these three Hamiltonians can be reproduced by means of simple potentials, like those used in the elastic network model:

$$H_{prot-x} = \sum_{j=1}^{x}\sum_{i=1}^{N-r} K_{ij}^{x}\left(\vec{D}_{ij} - \vec{D}_{ij}^{0}\right)^2 \qquad \text{(S24)}$$

$$H_x = \sum_{i,j=1}^{x} L_{ij}^{x}\left(\vec{D}_{ij} - \vec{D}_{ij}^{0}\right)^2 \qquad \text{(S25)}$$

where x stands for *r* or s depending if the Hamiltonian is referred to removed or added atoms, $\overline{D}_{ij}$ is the vector between *i* and *j* and $\vec{D}_{ij}^{0}$ is the corresponding equilibrium distance; $K_{ij}^{x}$ is the protein-mutation site stiffness matrix (product of Kirchoff matrix with a spring constant of 10 Kcal/mol Å$^2$ and a cutoff radii of 8 Å), and $L_{ij}^{x}$ corresponds to the stiffness matrix for the x-atoms.

As above indicated, it is also part of the invention the use of the ensemble of structures for the perturbed macromolecular receptor obtained, for virtual screening purposes. Thus, because the application of the method of the invention results in the generation of a new ensemble of perturbed structures of the receptor, this ensemble can subsequently be used in the *in silico* screening of large compound databases, in search of new hits for the receptor studied.

*In silico* virtual screening of large compound libraries in presence of the receptor structure relies on docking calculations. Typically, the receptor from an X-ray structure is refined and taken as a rigid particle in the filtering of organic compound databases in the search for novel hits. The virtual screening programs take each compound of the database in turn, optimize its interaction with the receptor by finding the best pose inside the active site by using a fitness function, and later on estimate the binding energy by using certain scoring functions. In this process, most programs explore not only the translational and rotational degrees of freedom of the small molecules, but also their conformational degrees of freedom by exploring all rotatable bonds. However, the receptor is taken as rigid, because of the heavy computational burden that imposes the flexibility of the receptor (thousands of degrees of freedom). The final result is that all the compounds of the database are ranked according to their estimated binding energies, and the top n% of the library is theoretically enriched with active compounds as compared to the rest of the library. Theoretical methods that increase the enrichment of the top n% of the database with actives are taken as superior to those that do not lead to such enrichment.

Although docking is routinely used in search of hits, the deficiencies in the scoring functions lead to a high false positive rate in the top scorers, and the rigid body approximation, by which the receptor is taken as an unmoving particle, leads to the appearance of false negatives, i.e. active molecules incorrectly estimated as inactive because of not being able to bind to the receptor as it is rigidly modelled. Because the active site residues rearrange differently to the presence of different molecules, some configurations of the active site are pre-arranged for binding some, but not all molecules. The only way that this can be overcome is by using not one but several images of the receptor, that is, by applying ensemble docking. In principle, the richer the ensemble used, the higher the probability of picking the right binding mode for more and more chemical scaffolds represented in the compound library.

Example 1 of the invention illustrates that the application of the method of the invention in virtual screening

gives superior results to a single crystal structure or an ensemble of structures of the receptors coming from a standard MD simulation.

Further, although the embodiments of the invention comprise processes performed in computer apparatus, the invention also extends to computer apparatus and to computer programs, particularly computer programs on or in a carrier, adapted for putting the invention into practice.

Accordingly, it also forms part of the invention a computer program product comprising program instructions for causing a computer to perform the method of molecular modelling for generating alternative, perturbed structures of a macromolecular receptor as defined above

The program may be in the form of source code, object code, a code intermediate source and object code such as in partially compiled form, or in any other form suitable for use in the implementation of the processes according to the invention.

In a preferred embodiment, the computer program product is embodied on a storage medium.

In another preferred embodiment, the computer program product is carried on a carrier signal. The carrier may be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means.

When the program is embodied in a signal that may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant process.

Finally, as mentioned above it also forms part of the invention a system of molecular modeling adapted for performing the method as defined in the first aspect of the invention, the system comprising: a) computer means for aligning the ensemble of structures of the unperturbed receptor on a common reference frame to eliminate the rotational and translational degrees of freedom of the receptor; b) computer means for diagonalizing the covariance matrix of the positional fluctuations built with the aligned ensemble of structures of the unperturbed receptor to obtain the eigenvectors and eigenvalues; c) computer means for defining a hybrid Hamiltonian of a perturbed state of the receptor in terms of a combination of the eigenvectors obtained in step b) plus a perturbation term in cartesian space that accounts for the difference between the unperturbed state and the perturbed state; d) computer means for defining the new forces acting on the receptor under the influence of the perturbation and integrating the equations of motion using Langevin or alternatively Brownian dynamics; and e) computer means for propagating the perturbed receptor to generate a trajectory and extracting a new ensemble of structures of the perturbed receptor.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

## EXAMPLES

### Example 1: Application of the method of the invention in virtual screening

In order to verify the validity of the drug-induced perturbation method in perturbed-EDMD, and its superiority in enhancing docking enrichment compared to single PDB (x-ray crystal structure) docking and ensemble docking on a standard MD ensemble, the inventors applied this method on eight protein systems for a comparative study. The goal was to see whether the application of the perturbed-EDMD method on proteins related to disease would generate ensembles that could yield superior results when compared to a single crystal structure or ensembles derived from standard MD simulations (which are the usual methods in the state of the art) in virtual screening campaigns. If results were superior for the perturbed-EDMD ensemble, it would mean the application of the method in *in silico* screening would be useful in search of new drugs.

The eight test protein systems were chosen based on two conditions. First, the proteins should have at least more than 12 known inhibitors and, secondly, their MD trajectories must be available in the MODEL database (Meyer T., et.al., "MODEL (Molecular Dynamics Extended Library): a database of atomistic molecular dynamics trajectories" Structure, 2010, vol. 18, pp. 1399-1409). The resulting systems were chosen and are listed in Table I. They represent systems that are under research in the pharmaceutical industry.

Table I: The 8 test protein systems chosen to validate the drug-induced perturbed-EDMD method.

| **Protein name** | **PDB id** | **Simulation time in MoDEL (nanoseconds)** |
|---|---|---|
| p38 MAP Kinase (ligand bound - holo) | 1A9U | 12ns |
| Cathepsin G (ligand bound - holo) | 1KYN | 10ns |
| Trypsin (ligand bound - holo) | 1DPO | 10ns |
| Serum albumin (apo) | 1UOR | 10ns |
| Carbonic anhydrase II (apo) | 12CA | 15ns |
| p38 MAP Kinase (apo) | 1A9U | 10ns |
| Ribonuclease A (apo) | 1A5P | 13ns |
| t-RNA transglycosylase (apo) | 1PUD | 10ns |

It should be noted that in the case of p38 MAP Kinase, the MODEL database contained MD trajectories for the protein, both in the presence and absence of the ligand SB2 (4-[5-(4-fluorophenyl)-2-[4[methylsulfinyl]phenyl]-3H-imidazol-4-yl]pyridine) for the crystal structure 1A9U. Both these trajectories were used in order to compare the extent of conformational sampling due to the effect of drug-induced perturbation in perturbed-EDMD on the open and closed forms of the same protein. Overall, there are five apo-protein and three holo-protein simulations.

The small molecule probe used to perturb the proteins' active sites through perturbed-EDMD was chosen based on its size and shape. A small Trypsin inhibitor was chosen (ligand from PDB id: 2JJC) as the perturbing probe for all the protein systems due to its relatively small size. Table II shows the structure of the probing drug molecule. This molecule was chosen because it was small enough to fit into all of the eight protein active sites.

Table II: The perturbation probe for the drug-induced perturbed-EDMD method

| Structure | PDB | Ligand name |
|---|---|---|
| 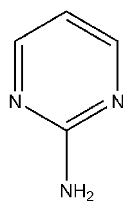 | 2JJC | PYRIMIDIN-2-AMINE |

The ligand datasets to be docked in the three situations (single crystal structure, ensemble coming from standard MD, or ensemble coming from perturbed-EDMD) were prepared in the following way. The actives were retrieved from the Protein Database (PDB database, www.rcsb.org) by selecting all available ligands from PDB complexes for each of the protein systems. This set of active compounds was mixed with diverse decoys (inactive molecules to test the methods). The decoy set consisted of 99 molecules from a pre-sorted drug-like set of compounds from a local database of 1.7 million compounds. The percentage of actives in each of the ligand datasets varied from ~11% to 50% depending on the protein. The final ligand dataset for each protein was prepared using Ligprep version 2.2 (Schrödinger, LLC, New York, NY, 2011) by generating low-energy ionization and tautomeric states within the range of pH 7.0±2.0. All ligands were energy-minimized using the OPLS-2005 force field implemented in MAESTROv8.5 (Schrödinger, LLC, New York, NY, 2011). The final result was a database that had actives and decoys (inactives) for all the proteins under study, and thus could be used in docking in the three situations tested to check which one discriminated actives from inactives more successfully in the top percentage of screened compounds.

The docking calculations in the three different situations were carried out in the following way:

*Single-structure* (*X-ray crystal structure) docking (for comparison)* - The ligand datasets were docked into the X-ray crystal structure conformation of their respective proteins using Glidev4.5 (Schrödinger, LLC, New York, NY, 2011), with the Glide SP (Standard Precision) scoring function. Docking was performed with all default settings of Glidev4.5. All hydrogens were added and the active site residues were protonated using the Protein Preparation Wizard of MAESTROv8.5. The receptor grid was constructed centered about the calculated centroid of the active site residues defined during grid generation.

*Molecular Dynamics generated snapshots and ensemble docking (for comparison)* - The MD trajectories of the

protein systems were converted to Cartesian co-ordinates and the 10,000 pdb frames were generated from the nanosecond-scale simulations. These frames were then clustered based on diverse heavy atom Root Mean Square Deviation (RMSD) using the kclust module of the 'Multiscale Modeling Tools for Structural Biology' (MMTSB) toolset (Michael Feig, John Karanicolas, Charles L. Brooks, III: MMTSB Tool Set, 2001, MMTSB NIH Research Resource, The Scripps Research Institute). In order to be consistent in the comparison with the perturbed-EDMD generated clusters, the inventors intended to generate 5 cluster bins, hence depending on the protein system, the appropriate radius was defined to generate exactly 5 clusters. The closest structure to the centroid structure in each of the cluster bins was selected as the cluster representative snapshot. In the end, 5 cluster representative snapshots were selected per protein system for MD ensemble docking. Respective ligand datasets were docked into each of the cluster representative snapshots of their protein. The individual ranked lists of the ligands were combined and the top ranking ligand conformation and score was retained. For example, if ligand A scored higher when docked in snapshot 1 compared to when docked in snapshot 2, the docked score of ligand A from snapshot 1 was retained. In this way, a sorted comprehensive rank based ensemble-docking ligand list was generated.

*Perturbed-EDMD generated snapshots and ensemble docking* - Each of the eight protein systems mentioned above in Table I was subject to the following workflow. In Step 1: PCA analysis- Starting from the pre-existing molecular dynamic (MD) simulation trajectories from the MODEL database, the eigenvectors and eigenvalues were calculated for only the active site residues (typically all residues within 5Å of the original bound ligand) and only the first 30 modes for each system were used for the perturbed-EDMD simulations. Step 2: Generation of initial probe co-ordinates -Inventors docked the small probe molecule found in Table II in the active site of each of the eight proteins (using Glidev4.5) to get the starting co-ordinates for ensuing the perturbed-EDMD run. Step 3: Drug-induced perturbation in EDMD- Next, the drug-induced perturbation in EDMD simulations was conducted. Only 1 drug (2JJC) was used to perturb the protein active site at a temperature of 300K (hence the ensemble was named 1D-300K). Each of these simulations were run for 10^7 steps with a time-step of 1fs for the protein and 10fs for the drug molecules. Step 4: Clustering- 10,000 snapshots (Cartesian co-ordinates in PDB format) from step 3 for each protein system were written (PDB frames were written every 10^3 steps). These 10,000 PDB frames were clustered using K-means clustering module (kclust) of the MMTSB toolset based on RMSD mode using all heavy atoms and enabling least square fit (Isqfit). Different clustering radius was used for each of the systems, depending on the extent of conformational diversity explored by the individual protein system. The range of radius used for separating the cluster bins for each of the proteins varied from 2.5Å -0.95Å. The cluster representative snapshots were chosen from each of the cluster bins (closest structure to the centroid-structure of the bin) to form the perturbed-EDMD generated cluster-representative-based protein ensemble for the eight protein systems studied. Step 5: Ensemble Docking- Once the ensemble of diverse protein conformations for each protein system was available, inventors conducted an ensemble docking procedure. Ligand datasets (consisting of known actives and decoys) for their respective proteins were docked into each of the cluster representative snapshots using Glide v4.5, SP scoring function. A scoring function based ranked ligand list was generated for each of the docking runs. These lists were combined to one master list and the top ranking ligand conformation and score was retained for each of the ligands in the data set.

In all three situations, the protein receptors (active site residues) were prepared for docking by using the Protein Preparation Wizard facility of MAESTROv8.5. This step included hydrogen optimization and protonation. The receptor grid for each protein was built centered around the centroid coordinate of all the residues defined as the active site. No water molecules or metal ions were retained for any of the calculations.

The metrics for evaluating the success of one method over the other was based on enrichment plots. These plots highlight the early relative enrichment of true positives (TPs) in the docked ranked list of ligands versus inactives (decoys). Ideally, if an ensemble of structures is better than another, it is more effective at selecting a varied pool of true positives in the top % of docked and scored molecules. Because certain chemical scaffolds found in the active compounds require for the receptor to be in a certain conformation and other chemical scaffolds in yet another, the more varied the ensemble of structures is, the more likely it will be to pick the correct binding mode for a pool of actives with high chemical diversity.

Because in virtual screening only the top percentage of the selected compounds are usually examined, prioritized or bought from commercial libraries, inventors limited the analysis of enrichment in all the cases to the top 15% of the libraries in each case. So, for each of the eight proteins under study, inventors examined the top 15% in search of true actives. The results of this analysis can be found in FIG. 1 from (a) to (d). The TP count was divided into four bins, the top 1%, 5%, 10% and 15%.

Analysis of the results in FIG. 1 clearly show that for the three cases examined, single structure (quoted as singlePDB) (for comparison), MD ensemble (quoted as MD) (for comparison), and perturbed-EDMD (quoted as 1D-300K because the systems where perturbed with only 1 drug and simulated at the temperature of 300K), the results are remarkably superior for the perturbed-EDMD ensemble when compared with the other two strategies. Thus, out of the eight proteins examined, perturbed-EDMD performs better in 5 cases, that is 1A9U-bnd (bound), 1KYN, 1DPO, 1UOR and 1A9U-unbnd, and as good as single structure or MD ensemble in the 3 remaining cases, that is 12CA, 1A5P and 1PUD. For

instance, in the case of protein 1KYN (Cathepsin G), found in Fig.1(a), the percentage of true positives in the 0.1 bin (10% of the database screened) is above 40% for the singlePDB strategy, above 30% for the MD ensemble, but above 60% in case of the perturbed-EDMD ensemble. Thus, the application of the method disclosed herein is superior to the other two in terms of enrichment for this target. Therefore, if a campaign in search for new active chemical scaffolds was undertaken for this disease-related enzyme, the application of the perturbed-EDMD method would give superior results to the other two approaches tested.
As a second example, in the case 1UOR (serum albumin), found in Fig. 1(b), the percentage enrichment is superior for the 1D-300K method, in all the bins analyzed, to singlePDB or MD. Thus, the percentage of true positives in the 0.1 bin (10% of the database screened) is barely above 40% for singlePDB, nearly 60% for the MD ensemble, but clearly above 70% for the perturbed-EDMD ensemble. This means that, in case of carrying out a virtual screening with this protein, the likelihood of retrieving more true positives (actives) in the top % of ranked compounds would be higher if perturbed-EDMD was used.

To further illustrate this example, in FIG. 2 the docking poses (binding modes) of warfarin, an anticoagulant drug, to serum albumin are found for a snapshot taken from the perturbed-EDMD ensemble and a second snapshot taken from the MD ensemble. The 1UOR structure of serum albumin is a challenging example for testing the methods. It is an apo structure, i.e. the protein in this structure solved by X-ray was crystallized in the absence of any binding molecules. Its binding site is buried, and slightly collapsed, so the docking of small molecules to its binding site is *a priori* very difficult due to steric clashes.

Thus, docking on the singlePDB crystal structure should not be very productive, as is seen in the Fig1(b) graph for singlePDB. However, the generation of an ensemble should give access to other configurations of the binding site that hopefully would turn out to be more productive than the single crystal structure. The results that were obtained clearly pointed out to binding modes that were not sterically hindered in the snapshots coming from perturbed-EDMD (see FIG. 2(a)), which explain the better performance of the method. However, in the case of snapshots taken from the MD ensemble, there was a steric clash that probably led to a worse performance in virtual screening (pointed out by the arrow in Fig2(b)).

As a third example, in the case 1A9U in its bound form (1A9U-bnd), it was also found that the perturbed-EDMD ensemble performed better than the other two strategies (FIG. 1(a)). For illustration purposes, FIG. 3(a) depicts the highest ranking binding mode found for the MD ensemble for one of the active compounds considered for this target, while FIG. 3(b) depicts the highest ranking binding mode for the same active for the perturbed-EDMD pool of structures. As it can be seen, in the former there are no favourable interactions with the receptor, while in the latter it was found that the highest ranking binding mode successfully reproduced the binding mode found experimentally for that particular active compound.

REFERENCES CITED IN THE APPLICATION

Teague SJ. "Implications of protein flexibility for drug discovery". Nat. Rev. Drug Disc. 2003, vol. 2, pp. 527-541.

Davis AM., et. al. "Application and limitations of X-ray crystallographic data in structure-based ligand and drug design" Angew. Chem. 2003, vol. 42, pp. 2718-2736.

Karplus M., et. al. "Molecular dynamics and protein function". PNAS 2005, vol. 102, pp. 6679-6685.

Rueda M., et. al. "Thorough validation of protein normal mode analysis: Comparative study with essential dynamics". Structure 2007, vol. 15, pp. 565-575.

Meyer T., et. al. "Essential dynamics: A tool for efficient trajectory compression and management". J. Chem. Theory Comput. 2006, vol. 2, pp. 251-258.

Amadei A. et. al. "An efficient method for sampling the essential subspace of proteins". J. Biomol. Str.Dyn. 1996, vol. 13, pp. 615-625.

Meyer T., et.al., "MODEL (Molecular Dynamics Extended Library): a database of atomistic molecular dynamics trajectories" Structure, 2010, vol. 18, pp. 1399-1409

Mehler EL., et. al. "Electrostatic effects in proteins: comparison of dielectric and charge models" Protein Eng. 1991, vol. 4, pp. 903-910.

Lazaridis, T. et. al. "Effective energy function for proteins in solution" Proteins 1999, vol. 35, pp. 133-152.

Arcangeli C. et al., "Molecular dynamics simulation and essential dynamics study of mutated plastocyanin: structural, dynamical and functional effects of a disulfide bridge insertion at the protein surface", Biophysical Chemistry 2001, vol. 92, no. 3, pp. 183-199.

Huang S. Y. et al., "Ensemble Docking of Multiple Protein Structures: Considering Protein Structural Variations in Molecular Docking" 2007, PROTEINS: Structure, Function, and Bioinformatics, vol. 66, pp. 399-421

## Claims

**1.** A computer-implemented method of molecular modeling for generating alternative, perturbed structures of a macromolecular receptor from an ensemble of structures of the unperturbed receptor, comprising:

a) aligning the ensemble of structures of the unperturbed receptor on a common reference frame to eliminate the rotational and translational degrees of freedom of the receptor;
b) diagonalizing the covariance matrix of the positional fluctuations built with the ensemble of structures of the unperturbed receptor to obtain the eigenvectors and eigenvalues;
c) defining a hybrid Hamiltonian of a perturbed state of the receptor in terms of a combination of the eigenvectors obtained in step b) plus a perturbation term in cartesian space (*V*) that accounts for the difference between the unperturbed state and the perturbed state

$$H = H^{(M)} + V$$

and;
d) defining the new forces acting on the receptor under the influence of the perturbation

$$\vec{F}_i^{(M)} = -\sum_{k=1}^{M} \frac{k_B T e_k^i}{\lambda_k} \left(\Delta \vec{r} \cdot \hat{e}_i\right) + \vec{F}_i^*$$

where $k_B$ is Boltzmann's constant, *T* is the absolute temperature, and where the sum on the right hand side is the resulting force derived from the Hamiltonian $H^{(M)}$, where $e_k^i$ is the component *i* of the eigenvector corresponding to mode *k*, and integrating the equations of motion using Langevin or, alternatively, Brownian dynamics; and
e) propagating the perturbed receptor to generate a trajectory and extracting a new ensemble of structures of the perturbed receptor.

**2.** The method of molecular modeling according to claim 1, comprising:

a) aligning the ensemble of structures of the unperturbed receptor on a common reference frame to eliminate the rotational and translational degrees of freedom of the receptor, by superimposing snapshots taken of the same macromolecule onto the common reference frame;
b) diagonalizing the covariance matrix of the positional fluctuations built with the aligned ensemble of structures of the unperturbed receptor to obtain the eigenvectors and eigenvalues;
c) defining mathematically a hybrid Hamiltonian of a perturbed state of the receptor as a combination of: c1) the eigenvectors obtained in step b), and c2) a perturbation term in cartesian space that accounts for the difference between the unperturbed state and the perturbed state;
d) defining mathematically the resulting forces acting on the receptor under the influence of the perturbation term and integrating Newton's equations of motion using Langevin or, alternatively, Brownian dynamics; and
e) propagating the perturbed receptor by iteratively integrating Newton's equations of motion to generate a trajectory as in step d), and extracting a new ensemble of structures of the perturbed receptor.

**3.** The method according to claims 1-2, wherein the perturbation term corresponds to the addition of an extra molecule

binding on a certain point of the receptor.

**4.** The method according to claim 3, wherein the extra molecule is a protein.

**5.** The method according to claim 3, wherein the extra molecule is an organic molecule.

**6.** The method according to claims 1-2, wherein the perturbation term corresponds to the addition of one or several mutations on the receptor.

**7.** The method according to claims 1-2, wherein the perturbation term corresponds to the addition of post-translational modifications of the receptor.

**8.** The method according to claim 7, wherein the post-translational modification is the phosphorylation of one or more residues of the receptor.

**9.** The method according to any of the claims 1-8, further comprising a previous step wherein molecular dynamics simulations are used to obtain the ensemble of structures of the unperturbed receptor.

**10.** The method according to any of the claims 1-9, further comprising for virtual screening purposes the steps of:

f) docking each small molecule found in a database of small organic molecules to at least one structure of the ensemble of structures of the perturbed macromolecular receptor;
g) estimating the affinity of each docked small molecule to the at least one structure of the ensemble of structures of the perturbed macromolecular receptor by calculating the interaction energy between the docked small molecule and the receptor, or alternatively by calculating the binding enthalpy or alternatively by calculating the binding free energy; and
h) ranking all the small molecules found in the database of small organic molecules based on the results of step g).

**11.** The method according to claim 10, wherein the estimation of the affinity is carried out by calculating the binding free energy wherein the calculation comprises the use of a scoring function.

**12.** A computer program product comprising program instructions for causing a computer to perform the method of molecular modeling for generating alternative, perturbed structures of a macromolecular receptor as defined in any of claims 1 to 11.

**13.** The computer program product according to claim 12, embodied on a storage medium.

**14.** The computer program product according to claim 12, carried on a carrier signal, wherein the carrier signal is an electrical or optical carrier signal.

**15.** A system of molecular modeling adapted for performing the method as defined in claim 1, the system comprising:

a) computer means for aligning the ensemble of structures of the unperturbed receptor on a common reference frame to eliminate the rotational and translational degrees of freedom of the receptor;
b) computer means for diagonalizing the covariance matrix of the positional fluctuations built with the aligned ensemble of structures of the unperturbed receptor to obtain the eigenvectors and eigenvalues;
c) computer means for defining a hybrid Hamiltonian of a perturbed state of the receptor in terms of a combination of the eigenvectors obtained in step b) plus a perturbation term in cartesian space that accounts for the difference between the unperturbed state and the perturbed state;
d) computer means for defining the new forces acting on the receptor under the influence of the perturbation and integrating the equations of motion using Langevin or ,alternatively, Brownian dynamics; and
e) computer means for propagating the perturbed receptor to generate a trajectory and extracting a new ensemble of structures of the perturbed receptor.

**Patentansprüche**

**1.** Ein computerimplementiertes Verfahren der molekularen Modellierung zur Erstellung von alternativen, gestörten

Strukturen von einem makromolekularen Rezeptor aus einem Ensemble von Strukturen des ungestörten Rezeptors, umfassend:

a) ausrichten des Ensembles von Strukturen des ungestörten Rezeptors auf einem gemeinsamen Bezugsrahmen, um die Freiheitsgrade entsprechend der Rotation und der Translation des Rezeptors zu entfernen;
b) diagonalisieren der Kovarianzmatrix der Lageschwankungen, die anhand von dem Ensemble von Strukturen des ungestörten Rezeptors erstellt worden ist, um die Eigenvektoren und Eigenwerte zu erhalten;
c) definieren von einem hybriden Hamiltonoperator eines gestörten Zustands des Rezeptors bezüglich einer Kombination von den in Schritt b) erhaltenen Eigenvektoren und einem Störungsterm im kartesischen Raum (*V*), der die Differenz zwischen dem ungestörten Zustand und dem gestörten Zustand darstellt

$$H = H^{(M)} + V$$

und;
d) definieren der neuen Kräfte, die auf den Rezeptor unter dem Einfluss der Störung wirken

$$\vec{F}_i^{(M)} = -\sum_{k=1}^{M} \frac{k_B T e_k^i}{\lambda_k} \left(\Delta\vec{r} \cdot \hat{e}_i\right) + \vec{F}_i^*$$

worin $k_B$ die Boltzmann-Konstante ist, $T$ die absolute Temperatur ist, und worin die Summe auf der rechten Seite die aus dem Hamiltonoperator $H^{(M)}$ abgeleitete resultierende Kraft ist, worin $e^i_k$ die Komponente $i$ des Eigenvektors entsprechend der Mode $k$ ist, und integrieren der Bewegungsgleichungen mittels Langevinscher oder, ersatzweise, Brownscher Dynamik; und
e) verbreiten des gestörten Rezeptors, um eine Trajektorie zu erzeugen und entnehmen von einem neuen Ensemble von Strukturen des gestörten Rezeptors.

**2.** Das Verfahren der molekularen Modellierung nach Anspruch 1, umfassend:

a) ausrichten des Ensembles von Strukturen des ungestörten Rezeptors auf einem gemeinsamen Bezugsrahmen, um die Freiheitsgrade entsprechend der Rotation und der Translation des Rezeptors zu entfernen, indem Momentaufnahmen des gleichen Moleküls auf dem gemeinsamen Bezugsrahmen überlagert werden;
b) diagonalisieren der Kovarianzmatrix der Lageschwankungen, die anhand von dem Ensemble von Strukturen des ungestörten Rezeptors erstellt worden ist, um die Eigenvektoren und Eigenwerte zu erhalten;
c) definieren von einem hybriden Hamiltonoperator eines gestörten Zustands des Rezeptors bezüglich einer Kombination von: c1) den in Schritt b) erhaltenen Eigenvektoren, und c2) einem Störungsterm im kartesischen Raum, der die Differenz zwischen dem ungestörten Zustand und dem gestörten Zustand darstellt;
d) definieren mittels mathematischer Methoden der resultierenden Kräfte, die auf den Rezeptor unter dem Einfluss vom Störungsterm wirken und integrieren der Newtonschen Bewegungsgleichungen mittels Langevinscher oder, ersatzweise, Brownscher Dynamik; und
e) verbreiten des gestörten Rezeptors durch iterative Integration der Newtonschen Bewegungsgleichungen, um eine Trajektorie wie in Schritt d) zu erzeugen, und entnehmen von einem neuen Ensemble von Strukturen des gestörten Rezeptors.

**3.** Das Verfahren nach den Ansprüchen 1-2, wobei der Störungsterm der Zugabe von einem zusätzlichen Molekül entspricht, das sich an einer bestimmten Stelle des Rezeptors bindet.

**4.** Das Verfahren nach Anspruch 3, wobei das zusätzliche Molekül ein Protein ist.

**5.** Das Verfahren nach Anspruch 3, wobei das zusätzliche Molekül ein organisches Molekül ist.

**6.** Das Verfahren nach den Ansprüchen 1-2, wobei der Störungsterm der Zugabe von einer oder mehreren Mutationen auf dem Rezeptor entspricht.

**7.** Das Verfahren nach den Ansprüchen 1-2, wobei der Störungsterm der Zugabe von posttranslationalen Modifikationen des Rezeptors entspricht.

8. Das Verfahren nach Anspruch 7, wobei die posttranslationale Modifikation die Phosphorylierung von einem oder mehreren Resten des Rezeptors ist.

9. Das Verfahren nach einem der Ansprüche 1-8, weiterhin umfassend einen vorherigen Schritt, in dem auf molekularer Dynamik beruhende Simulationen verwendet werden, um das Ensemble von Strukturen des ungestörten Rezeptors zu erhalten.

10. Das Verfahren nach einem der Ansprüche 1-9, weiterhin umfassend die folgenden Schritte für Zwecke virtuellen Screenings:

f) durchführen eines *Dockings* von jedem kleinen in einer Datenbank von kleinen organischen Molekülen gefundenen Molekül mit mindestens einer Struktur des Ensembles von Strukturen des gestörten makromolekularen Rezeptors;
g) abschätzen der Affinität von jedem dem *Docking* ausgesetzten kleinen Molekül mit der mindestens einen Struktur des Ensembles von Strukturen des gestörten makromolekularen Rezeptors, indem die Wechselwirkungsenergie zwischen dem *Docking* ausgesetzten kleinen Molekül und dem Rezeptor berechnet wird oder, ersatzweise, indem die Bindungsenthalpie berechnet wird oder, ersatzweise, indem die freie Bindungsenergie berechnet wird; und
h) einordnen von allen kleinen in der Datenbank von kleinen organischen Molekülen gefundenen Molekülen basierend auf den Ergebnissen des Schritts g).

11. Das Verfahren nach Anspruch 10, wobei die Abschätzung der Affinität durch die Berechnung der freien Bindungsenergie durchgeführt wird, wobei die Berechnung die Verwendung von einer Bewertungsfunktion umfasst.

12. Ein Computerprogrammprodukt umfassend Programmanweisungen zur Veranlassung der Durchführung des Verfahrens der molekularen Modellierung zur Erstellung von alternativen, gestörten Strukturen von einem makromolekularen Rezeptor wie in einem der Ansprüche 1 bis 11 definiert durch einen Computer.

13. Das Computerprogrammprodukt nach Anspruch 12, das in einem Speichermedium enthalten ist.

14. Das Computerprogrammprodukt nach Anspruch 12, das in einem Trägersignal getragen ist, wobei das Trägersignal ein elektrisches oder ein optisches Trägersignal ist.

15. Ein System der molekularen Modellierung, das angepasst für die Durchführung des Verfahrens wie in Anspruch 1 definiert ist, wobei das System folgendes umfasst:

a) ein Computermittel zur Ausrichtung des Ensembles von Strukturen des ungestörten Rezeptors auf einem gemeinsamen Bezugsrahmen, um die Freiheitsgrade entsprechend der Rotation und der Translation des Rezeptors zu entfernen;
b) ein Computermittel zur Diagonalisierung der Kovarianzmatrix der Lageschwankungen, die anhand von dem ausgerichteten Ensemble von Strukturen des ungestörten Rezeptors erstellt worden ist, um die Eigenvektoren und Eigenwerte zu erhalten;
c) ein Computermittel zur Definition von einem hybriden Hamiltonoperator eines gestörten Zustands des Rezeptors bezogen auf eine Kombination von den in Schritt b) erhaltenen Eigenvektoren und einem Störungsterm im kartesianischen Raum, der die Differenz zwischen dem ungestörten Zustand und dem gestörten Zustand darstellt;
d) ein Computermittel zur Definition der neuen Kräfte, die auf den Rezeptor unter dem Einfluss der Störung wirken und Integration der Bewegungsgleichungen mittels Langevinscher oder, ersatzweise, Brownscher Dynamik; und
e) ein Computermittel zur Verbreitung des gestörten Rezeptors, um eine Trajektorie zu erzeugen und Entnahme von einem neuen Ensemble von Strukturen des gestörten Rezeptors.

## Revendications

1. Un procédé de modélisation moléculaire implémenté par ordinateur pour générer des structures perturbées alternatives d'un récepteur moléculaire à partir d'un ensemble de structures du récepteur non perturbé, comprenant :

a) aligner l'ensemble de structures du récepteur non perturbé sur un cadre de référence commun pour éliminer les degrés de liberté de rotation et de translation du récepteur ;
b) diagonaliser la matrice de covariance des fluctuations de position formée avec l'ensemble de structures du récepteur non perturbé pour obtenir les vecteurs propres et les valeurs propres ;
c) définir un hamiltonien hybride d'un état perturbé du récepteur en termes d'une combinaison des vecteurs propres obtenus dans l'étape b) et un terme de perturbation dans un espace cartésien (*V*) qui représente la différence entre l'état non perturbé et l'état perturbé

$$H = H^{(M)} + V$$

et ;
d) définir les nouvelles forces agissant sur le récepteur sous l'influence de la perturbation

$$\vec{F}_i^{(M)} = -\sum_{k=1}^{M} \frac{k_B T e_k^i}{\lambda_k} \left(\Delta\vec{r} \cdot \hat{e}_i\right) + \vec{F}_i^*$$

où $k_B$ est la constante de Boltzmann, *T* est la température absolue, et où la somme du côté droit est la force résultante obtenue à partir du hamiltonien $H^{(M)}$, où $e^i_k$ est la composante *i* du vecteur propre correspondant au mode *k*, et intégrer les équations de mouvement moyennant dynamique de Langevin ou, alternativement, dynamique de Brown ; et
e) propager le récepteur perturbé afin de générer une trajectoire et extraire un nouvel ensemble de structures du récepteur perturbé.

**2.** Le procédé de modélisation moléculaire selon la revendication 1, comprenant:

a) aligner l'ensemble de structures du récepteur non perturbé sur un cadre de référence commun afin d'éliminer les degrés de liberté de rotation et de translation du récepteur, en superposant des instantanés pris de la même macromolécule sur le cadre de référence commun ;
b) diagonaliser la matrice de covariance des fluctuations de position formée avec l'ensemble aligné de structures du récepteur non perturbé afin d'obtenir les vecteurs propres et les valeurs propres ;
c) définir mathématiquement un hamiltonien hybride d'un état perturbé du récepteur comme une combinaison de : c1) les vecteurs propres obtenus dans l'étape b), et c2) un terme de perturbation dans un espace cartésien qui représente la différence entre l'état non perturbé et l'état perturbé ;
d) définir mathématiquement les forces résultantes agissant sur le récepteur sous l'influence du terme de perturbation et intégrer les équations de mouvement de Newton moyennant dynamique de Langevin ou, alternativement, dynamique de Brown ; et
e) propager le récepteur perturbé en intégrant de façon itérative les équations de mouvement de Newton afin de générer une trajectoire comme dans l'étape d), et extraire un nouvel ensemble de structures du récepteur perturbé.

**3.** Le procédé selon les revendications 1-2, dans lequel le terme de perturbation correspond à l'addition d'une molécule additionnelle se liant sur un certain point du récepteur.

**4.** Le procédé selon la revendication 3, dans lequel la molécule additionnelle est une protéine.

**5.** Le procédé selon la revendication 3, dans lequel la molécule additionnelle est une molécule organique.

**6.** Le procédé selon les revendications 1-2, dans lequel le terme de perturbation correspond à l'addition d'une ou de quelques mutations sur le récepteur.

**7.** Le procédé selon les revendications 1-2, dans lequel le terme de perturbation correspond à l'addition de modifications post-traductionnelles du récepteur.

**8.** Le procédé selon la revendication 7, dans lequel la modification post-traductionnelle est la phosphorylation d'un ou de plusieurs restes du récepteur.

**9.** Le procédé selon l'une quelconque des revendications 1-8, comprenant en outre une étape précédente dans laquelle des simulations de dynamique moléculaire sont utilisées pour obtenir un ensemble de structures du récepteur non perturbé.

**10.** Le procédé selon l'une quelconque des revendications 1-9, comprenant en outre aux fins de criblage virtuel les étapes de :

f) soumettre à amarrage chaque molécule petite trouvée dans une base de données de molécules organiques petites vers au moins une structure de l'ensemble de structures du récepteur macromoléculaire perturbé ;
g) estimer l'affinité de chaque molécule petite soumise à amarrage avec l'au moins une structure de l'ensemble de structures du récepteur macromoléculaire perturbé en calculant l'énergie d'interaction entre la molécule petite soumise à amarrage et le récepteur ou, alternativement, en calculant l'enthalpie de liaison ou, alternativement, en calculant l'énergie libre de liaison ; et
h) classer toutes les molécules petites trouvées dans la base de données de molécules organiques petites à partir des résultats de l'étape g).

**11.** Le procédé selon la revendication 10, dans lequel l'estimation de l'affinité est effectuée en calculant l'énergie libre de liaison, où le calcule comprend l'utilisation d'une fonction de score.

**12.** Un produit de programme informatique comprenant des instructions de programme pour faire qu'un ordinateur effectue le procédé de modélisation moléculaire pour générer des structures perturbées alternatives d'un récepteur moléculaire tel que défini dans l'une quelconque des revendications 1 à 11.

**13.** Le produit de programme informatique selon la revendication 12, incorporé dans un support de stockage.

**14.** Le produit de programme informatique selon la revendication 12, porté dans un signal porteur, dans lequel le signal porteur est un signal porteur électrique ou un signal porteur optique.

**15.** Un système de modélisation moléculaire adapté pour exécuter le procédé tel que défini dans la revendication 1, le système comprenant :

a) un moyen informatique pour aligner l'ensemble de structures du récepteur non perturbé sur un cadre de référence commun afin d'éliminer les degrés de liberté de rotation et de translation du récepteur ;
b) un moyen informatique pour diagonaliser la matrice de covariance des fluctuations de position formée avec l'ensemble aligné de structures du récepteur non perturbé afin d'obtenir les vecteurs propres et les valeurs propres;
c) un moyen informatique pour définir un hamiltonien hybride d'un état perturbé du récepteur en termes d'une combinaison des vecteurs propres obtenus dans l'étape b) et un terme de perturbation dans un espace cartésien qui représente la différence entre l'état non perturbé et l'état perturbé ;
d) un moyen informatique pour définir les nouvelles forces agissant sur le récepteur sous l'influence de la perturbation et intégrer les équations de mouvement moyennant dynamique de Langevin ou, alternativement, dynamique de Brown ; et
e) un moyen informatique pour propager le récepteur perturbé afin de générer une trajectoire et extraire un nouvel ensemble de structures du récepteur perturbé.

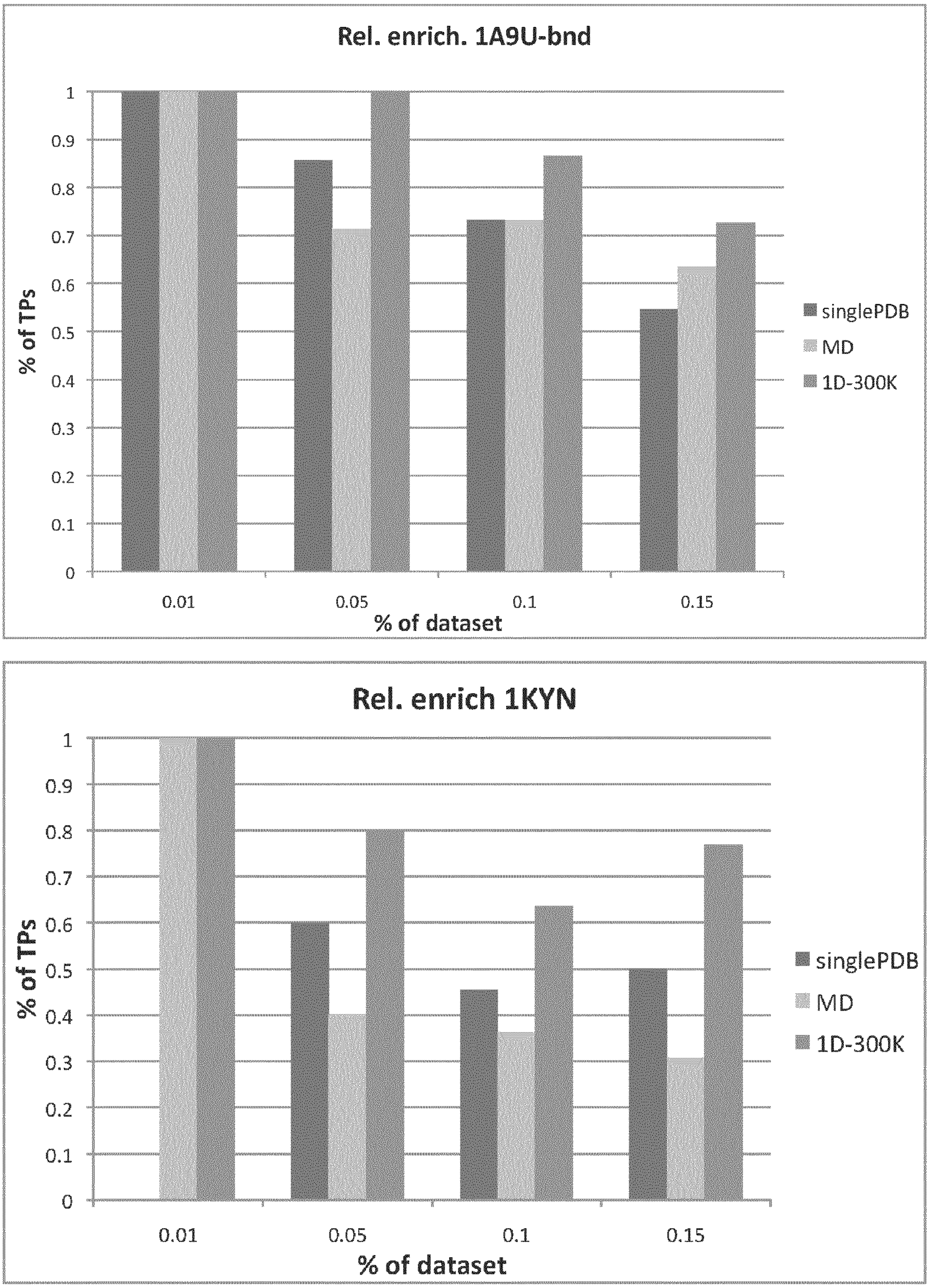
Rel. enrich. 1A9U-bnd
% of TPs
% of dataset
0.01
0.05
0.1
0.15
singlePDB
MD
1D-300K
Rel. enrich 1KYN
% of TPs
% of dataset
singlePDB
MD
1D-300K

FIG. 1(a)

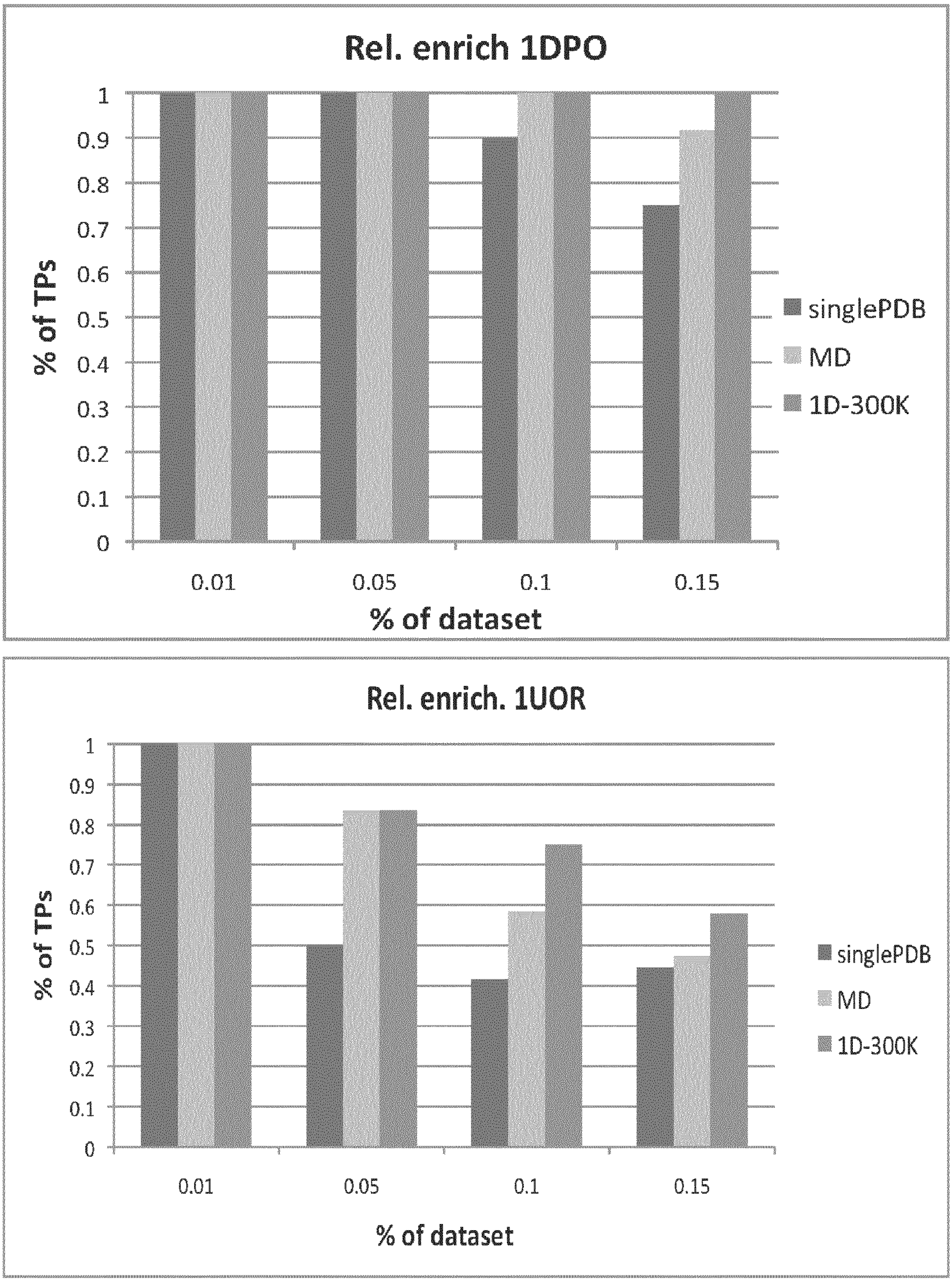
Rel. enrich 1DPO
% of TPs
1
0.9
0.8
0.7
0.6
0.5
0.4
0.3
0.2
0.1
0
0.01
0.05
0.1
0.15
% of dataset
singlePDB
MD
1D-300K
Rel. enrich. 1UOR
% of TPs
1
0.9
0.8
0.7
0.6
0.5
0.4
0.3
0.2
0.1
0
0.01
0.05
0.1
0.15
% of dataset
singlePDB
MD
1D-300K

FIG. 1(b)

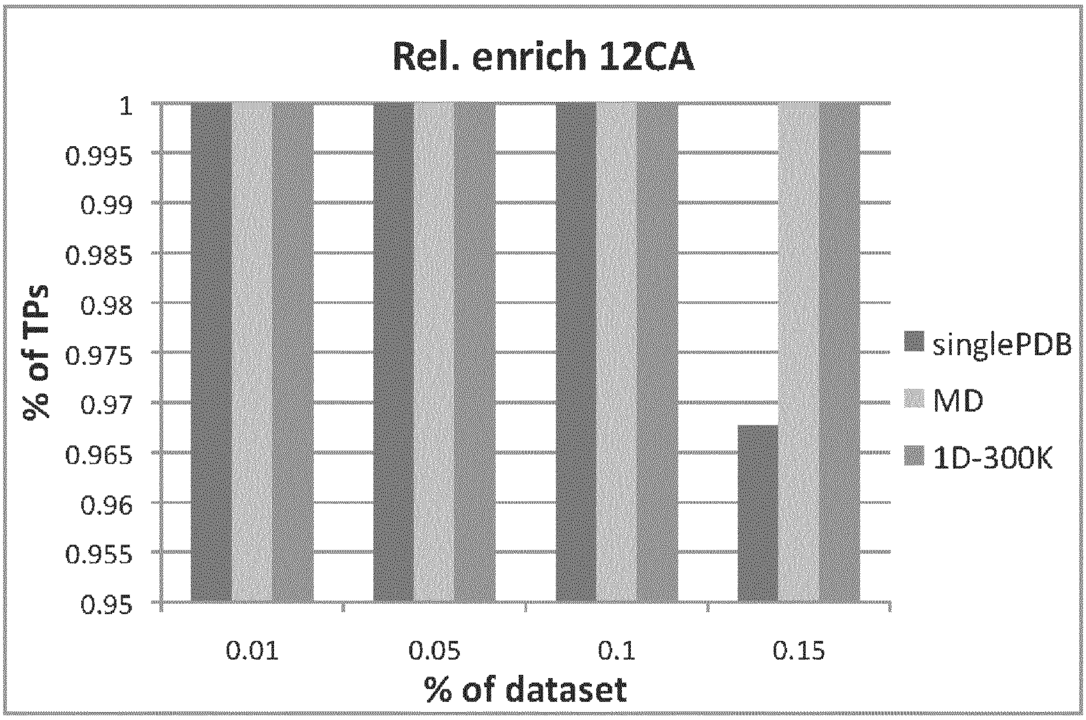
Rel. enrich 12CA
% of TPs
1
0.995
0.99
0.985
0.98
0.975
0.97
0.965
0.96
0.955
0.95
0.01
0.05
0.1
0.15
% of dataset
singlePDB
MD
1D-300K

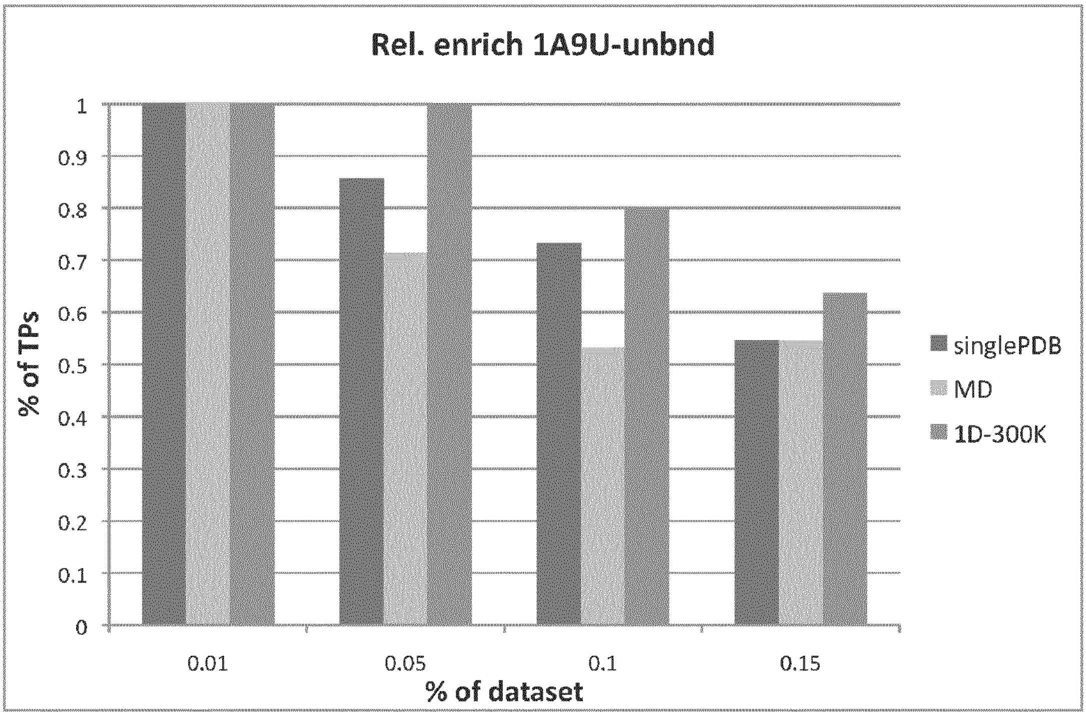
Rel. enrich 1A9U-unbnd
% of TPs
1
0.9
0.8
0.7
0.6
0.5
0.4
0.3
0.2
0.1
0
0.01
0.05
0.1
0.15
% of dataset
singlePDB
MD
1D-300K

FIG. 1(c)

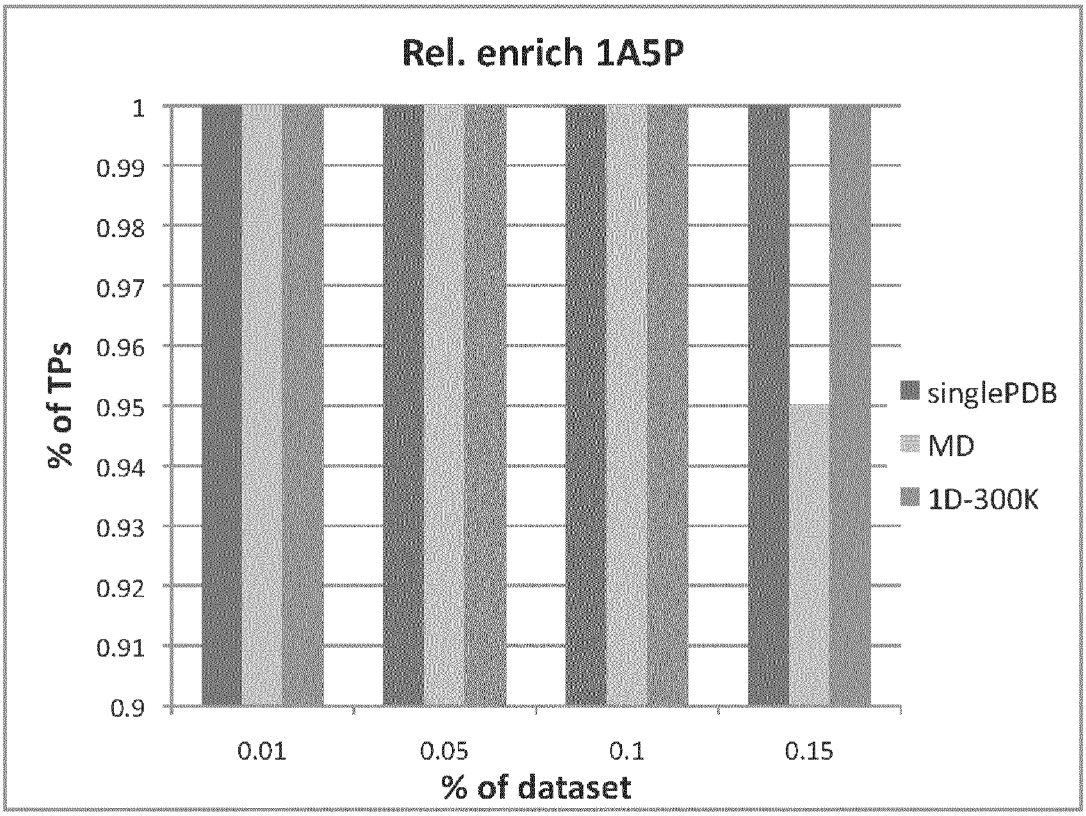
Rel. enrich 1A5P
% of TPs
1
0.99
0.98
0.97
0.96
0.95
0.94
0.93
0.92
0.91
0.9
0.01
0.05
0.1
0.15
% of dataset
singlePDB
MD
1D-300K

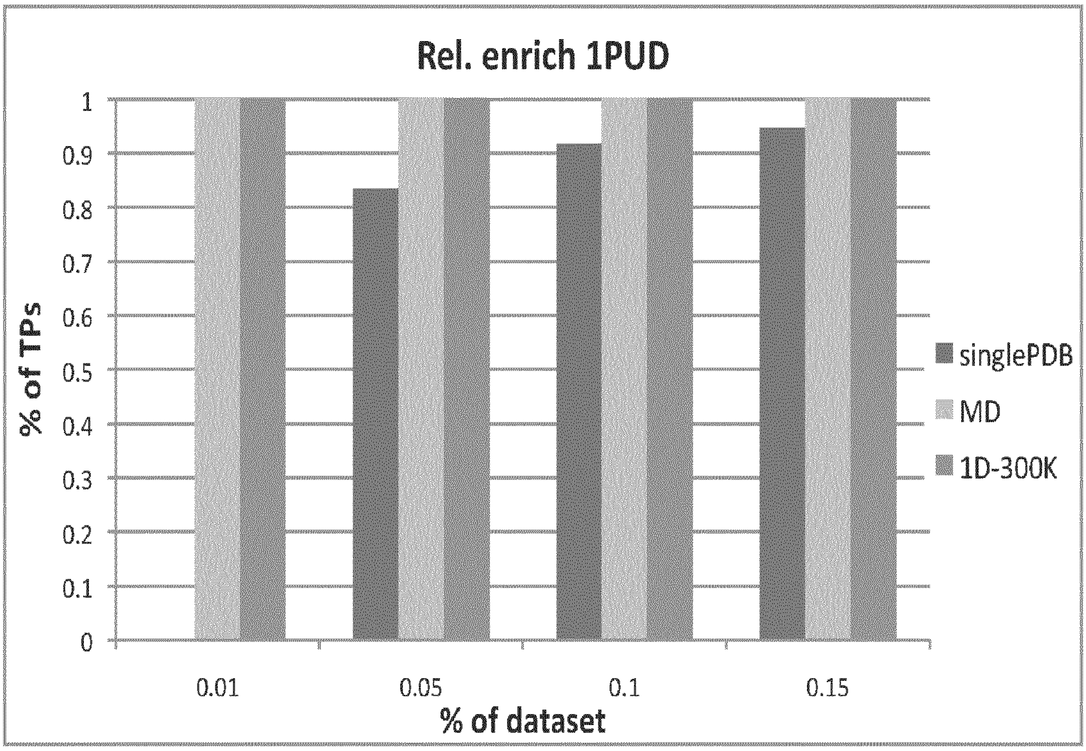
Rel. enrich 1PUD
% of TPs
1
0.9
0.8
0.7
0.6
0.5
0.4
0.3
0.2
0.1
0
0.01
0.05
0.1
0.15
% of dataset
singlePDB
MD
1D-300K

FIG. 1(d)

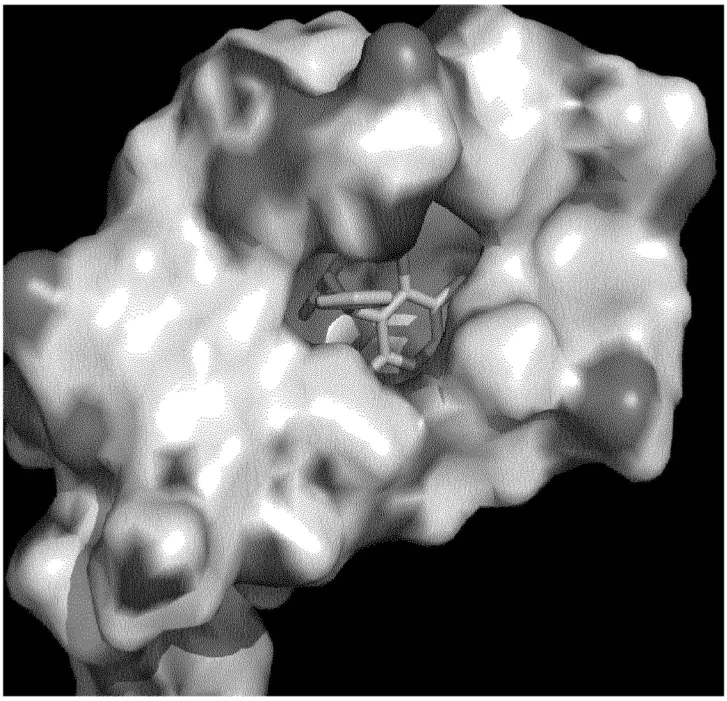

FIG. 2(a)

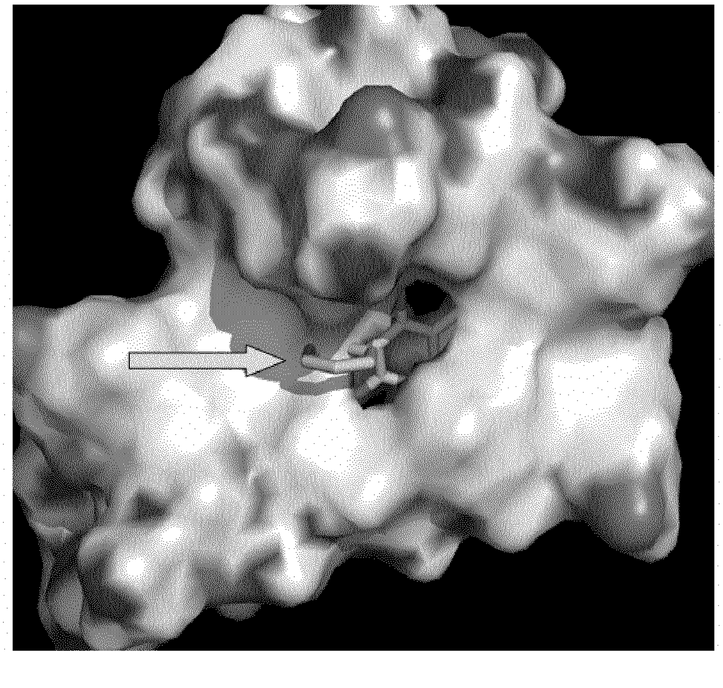

FIG. 2(b)

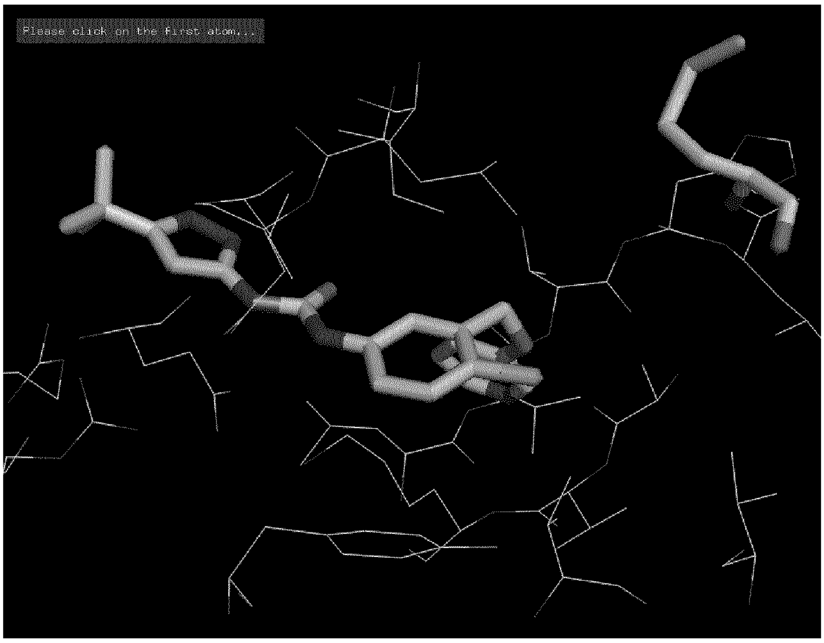
Please click on the first atom...

FIG. 3(a)

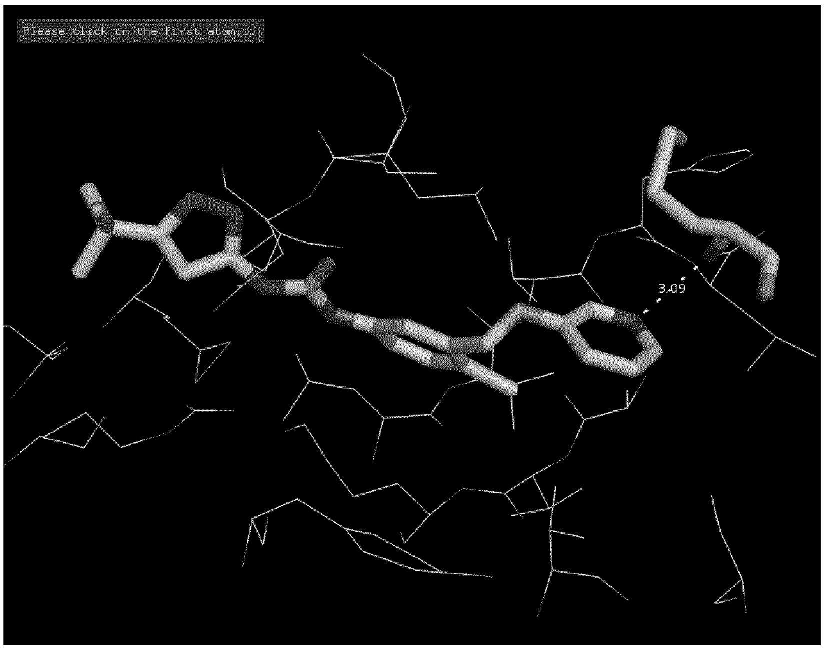
Please click on the first atom...
3.09

FIG. 3(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TEAGUE SJ.** Implications of protein flexibility for drug discovery. *Nat. Rev. Drug Disc.,* 2003, vol. 2, 527-541
- **DAVIS AM.** Application and limitations of X-ray crystallographic data in structure-based ligand and drug design. *Anqew. Chem.,* 2003, vol. 42, 2718-2736
- **KARPLUS M.** Molecular dynamics and protein function. *Proc. of the Nat. Acad. of Sciences,* 2005, vol. 102, 6679-6685
- **RUEDA M.** Thorough validation of protein normal mode analysis: Comparative study with essential dynamics. *Structure,* 2007, vol. 15, 565-575
- **MEYER T.** Essential dynamics: A tool for efficient trajectory compression and management. *J. Chem. Theory Comput.,* 2006, vol. 2, 251-258
- **AMADEI A.** An efficient method for sampling the essential subspace of proteins. *J. Biomol. Str. Dyn.,* 1996, vol. 13, 615-625
- **ARCANGELI C. et al.** Molecular dynamics simulation and essential dynamics study of mutated plastocyanin: structural, dynamical and functional effects of a disulfide bridge insertion at the protein surface. *Biophysical Chemistry,* 2001, vol. 92, 183-199
- **HUANG S. Y. et al.** Ensemble Docking of Multiple Protein Structures: Considering Protein Structural Variations in Molecular Docking. *PROTEINS: Structure, Function, and Bioinformatics,* 2007, vol. 66, 399-421
- **MEHLER EL.** Electrostatic effects in proteins: comparison of dielectric and charge models. *Protein Eng.,* 1991, vol. 4, 903-910
- **LAZARIDIS, T.** Effective energy function for proteins in solution. *Proteins,* 1999, vol. 35, 133-152
- **MEYER T.** MODEL (Molecular Dynamics Extended Library): a database of atomistic molecular dynamics trajectories. *Structure,* 2010, vol. 18, 1399-1409
- **MICHAEL FEIG ; JOHN KARANICOLAS ; CHARLES L. BROOKS.** III: MMTSB Tool Set. MMTSB NIH Research Resource, The Scripps Research Institute, 2001
- **DAVIS AM.** Application and limitations of X-ray crystallographic data in structure-based ligand and drug design. *Angew. Chem.,* 2003, vol. 42, 2718-2736
- **KARPLUS M.** Molecular dynamics and protein function. *PNAS,* 2005, vol. 102, 6679-6685
- **AMADEI A.** An efficient method for sampling the essential subspace of proteins. *J. Biomol. Str.Dyn.,* 1996, vol. 13, 615-625
- **MEHLER EL.** Electrostatic effects in proteins: comparison of dielectric and charge models. *Protein Eng.,* 1991, vol. 4, 903-910
- **ARCANGELI C. et al.** Molecular dynamics simulation and essential dynamics study of mutated plastocyanin: structural, dynamical and functional effects of a disulfide bridge insertion at the protein surface. *Biophysical Chemistry,* 2001, vol. 92 (3), 183-199